(19) Europäisches Patentamt
European Patent Office
Office européen des brevets

(11) Numéro de publication : **0 053 537**
**B1**

# (12) FASCICULE DE BREVET EUROPÉEN

(45) Date de publication du fascicule du brevet : **25.07.84**

(51) Int. Cl.³ : **C 07 D501/24**

(21) Numéro de dépôt : **81401823.0**

(22) Date de dépôt : **19.11.81**

## (54) Nouveaux dérivés de vinyl-3 céphalosporines et leur préparation.

(30) Priorité : **20.11.80 FR 8024634**

(43) Date de publication de la demande : **09.06.82 Bulletin 82/23**

(45) Mention de la délivrance du brevet : **25.07.84 Bulletin 84/30**

(84) Etats contractants désignés : **AT BE CH DE FR GB IT LI LU NL SE**

(56) Documents cités :
**FR-A- 2 081 451**
**FR-A- 2 137 899**
**US-A- 4 065 620**
**J. Med. Chem. 18 (10), 986 (1975)**
**Chem. Ber. 101, 4048 (1968)**
**Tetrahedron, 35, 1675 (1979)**

(73) Titulaire : **RHONE-POULENC SANTE**
**Les Miroirs 18 Avenue d'Alsace**
**F-92400 Courbevoie Cedex (FR)**

(72) Inventeur : **Farge, Daniel**
**30 rue des Pins Sylvestres**
**F-94320 Thiais (FR)**
Inventeur : **Le Roy, Pierre**
**2 Allée des Cerisiers**
**F-94320 Thiais (FR)**
Inventeur : **Moutonnier, Claude**
**3 rue Auguste Rodin**
**F-92350 Le Plessis Robinson (FR)**
Inventeur : **Peyronel, Jean-François**
**36 parc d'Ardenay**
**F-91110 Palaiseau (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 053 537 B1

Jouve, 18, rue St-Denis, 75001 Paris, France

## Description

La présente invention concerne de nouvelles vinyl-3 céphalosporines de formule générale :

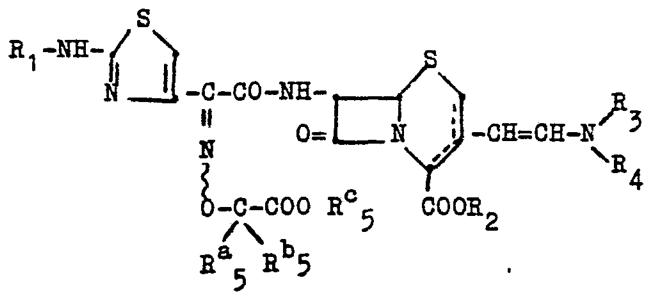

(I)

On connaît déjà des dérivés de vinyl-3 céphalosporines par les demandes de brevets français 2 081 451 et 2 137 899.

Les produits selon l'invention se distinguent par le fait que le groupement vinyle situé en position -3 porte lui-même un radical amino.

Dans la formule générale (I) les produits se présentent sous forme bicyclooctène-2 ou -3 (selon la nomenclature des Chemical Abstracts) ;

le substituant sur l'atome de carbone en position -3 du bicyclooctène présente la stéréomérie cis ou trans ;

le symbole $R_1$ représente un radical protecteur d'amine, par exemple un radical t.butoxycarbonyle, trichloro-2,2,2 éthoxycarbonyle trityle, benzyle, dibenzyle, benzyloxycarbonyle, p.nitrobenzyloxy-carbonyle, p.méthoxybenzyloxycarbonyle, formyle, chloracétyle, trichloracétyle ou trifluoracétyle ;

le symbole $R_2$ représente un radical méthoxyméthyle, t.butyle, benzhydryle, p.nitrobenzyle, p.méthoxybenzyle ou un radical facilement éliminable par voie enzymatique et de formule générale :

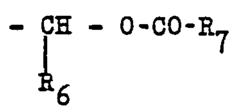

(II)

dans laquelle $R_6$ représente un atome d'hydrogène ou un radical alcoyle et $R_7$ représente un radical alcoyle ou cyclohexyle ;

les symboles $R_3$ et $R_4$, qui sont identiques ou différents, représentent des radicaux alcoyles (éventuellement substitués par un radical hydroxy, alcoyloxy, amino, alcoylamino ou dialcoylamino) ou phényle ou forment ensemble, avec l'atome d'azote auquel ils sont rattachés, un hétérocycle saturé à 5 ou 6 chaînons contenant éventuellement un autre hétéroatome choisi parmi l'azote, l'oxygène et le soufre et éventuellement substitué par un radical alcoyle ;

les symboles $R^a_5$ et $R^b_5$, identiques ou différents, sont choisis parmi les atomes d'hydrogène et les radicaux alcoyles ou forment ensemble un radical alcoylène contenant 2 ou 3 atomes de carbone et le symbole $R^c_5$ représente un radical protecteur d'acide, par exemple un radical méthoxyméthyle, t.butyle, benzhydryle, benzyle, nitrobenzyle ou p.méthoxybenzyle.

Il est entendu que, sauf mention spéciale, les portions ou radicaux alcoyles ou acyles sont droits ou ramifiés et contient 1 à 4 atomes de carbone.

Il est également entendu que les isomères bicyclooctène-2 et -3, cis (ou « Z ») et trans (ou « E ») ainsi que leurs mélanges entrent dans le cadre de l'invention.

Par ailleurs le groupement acyloxyimino du substituant en position -7 peut se trouver dans l'une des positions syn, formule (IIIa) ou anti, formule (IIIb) :

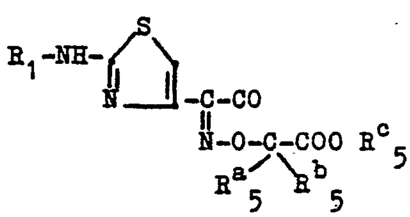

(IIIa)

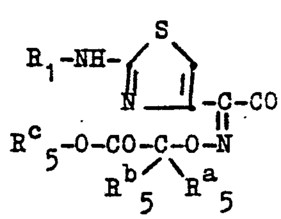

(IIIb)

et ces isomères et leurs mélanges entrent dans le cadre de l'invention.

De plus lorsque $R^a_5$ et $R^b_5$ sont différents, l'invention concerne les diastéréoisomères correspondants ainsi que leurs mélanges.

1. Selon l'invention, les produits de formule générale (I) pour lesquels $R_3$ et $R_4$ ont les définitions données précédemment, à l'exception de représenter alcoyle substitué par hydroxy, amino ou alcoylamino, peuvent être obtenus par action d'un produit, éventuellement préparé in situ, de formule générale :

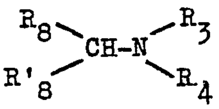

(IV)

[dans laquelle $R_3$ et $R_4$ sont définis comme précédemment et $R_8$ et $R'_8$ qui sont identiques ou différents, soit représentent des groupements de formule générale :

$$—X_2R_9 \quad (V)$$

dans laquelle $X_2$ est un atome d'oxygène et $R_9$ représente un radical alcoyle contenant 1 à 5 atomes de carbone, ou phényle soit représentent l'un un radical de formule générale (V) dans laquelle $X_2$ est oxygène ou soufre et l'autre un radical amino de formule générale :

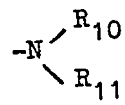

(VI)

dans laquelle $R_{10}$ et $R_{11}$ sont définis comme $R_3$ et $R_4$ dans la formule générale (IV), soit encore représentent chacun un radical de formule générale (VI)]
sur un dérivé de céphalosporine de formule générale :

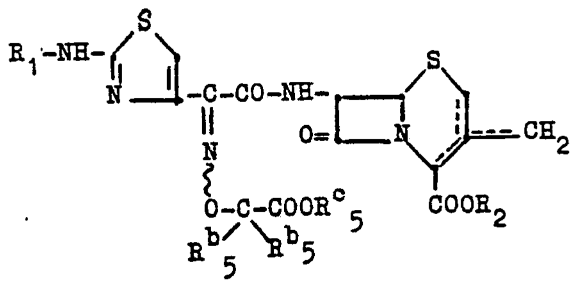

(VII)

dans laquelle, les divers symboles étant définis comme précédemment, le dérivé se présente sous forme méthyl-3 bicyclooctène-2 ou -3 ou méthylène-3 bicyclooctane.

On opère généralement dans un solvant organique tel que le diméthylformamide, l'hexaméthylphosphorotriamide, l'acéto-nitrile, le diméthylacétamide, l'acétate d'éthyle, le dioxanne ou un solvant chloré (par exemple le dichloro-1,2 éthane) ou dans un mélange de tels solvants à une température comprise entre 20 °C et la température de reflux du mélange réactionnel.

Lorsque l'on choisit un produit de formule générale (IV) dans laquelle le radical (VI) est différent de $—NR_3R_4$, il est préférable de choisir un tel produit de manière que l'amine HN $R_{10}R_{11}$ soit plus volatile que HN $R_3R_4$.

2. Dans une étape subséquente, les produits de formule générale (I) dans laquelle $R_3$ et $R_4$, qui sont identiques ou différents, représentent des radicaux alcoyle substitués par hydroxy, amino ou alcoylamino, et les autres symboles sont définis comme précédemment, peuvent être obtenus par transénamination à partir d'un produit de formule générale (I) dans laquelle $R_3$ et $R_4$ représentent des radicaux alcoyle, de préférence méthyle.

La réaction s'effectue par action d'une amine de formule générale :

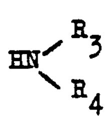

(VIII)

(dans laquelle $R_3$ et $R_4$ ont les définitions correspondantes) sur le produit de formule générale (I) et l'on opère dans des conditions analogues à celles décrites précédemment pour l'action d'un produit de formule générale (IV) sur un dérivé de formule générale (VII).

Les produits de formule générale (IV) peuvent être préparés selon les méthodes décrites par H. Bredereck et coll., Chem. Ber. 101, 41 (1968), Chem. Ber. 101, 3058 (1968) et Chem. Ber. 106, 3725 (1973).

Les dérivés de la céphalosporine de formule générale (VII) peuvent être préparés à partir des produits de formule générale :

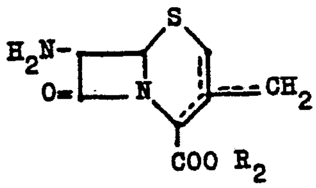

(IX)

[dans laquelle, $R_2$ étant défini comme précédemment, la position de la double liaison est définie comme pour le produit de formule générale (VII)] par action d'un acide de formule générale :

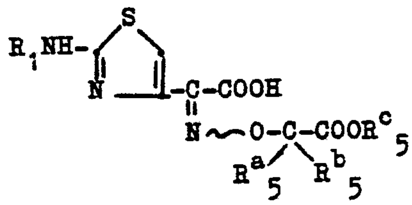

(X)

[dans laquelle les divers symboles sont définis comme précédemment] ou d'un dérivé réactif de cet acide. Il est entendu que l'acide de formule générale (X) sous forme syn, anti ou leurs mélanges, conduit respectivement aux produits de formule générale (VII) de forme syn, anti ou à leurs mélanges.

Généralement on effectue la condensation du produit de formule générale (X), dont la fonction acide est libre, sur l'amino-7 céphalosporine de formule générale (IX) dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre − 20 et 40 °C.

Lorsque l'on utilise un dérivé réactif de l'acide de formule générale (X), il est possible de mettre en œuvre l'anhydride, un anhydride mixte ou un ester réactif de formule générale :

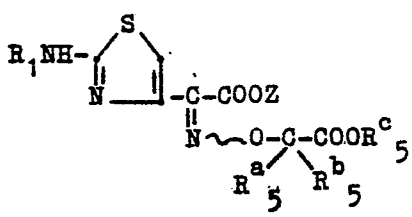

(XI)

[dans laquelle $R^a_5$, $R^b_5$, $R^c_5$ et $R_1$ étant définis comme ci-dessus, Z représente un radical succinimido, benzotriazolyle-1, nitro-4 phényle, dinitro-2,4 phényle, pentachlorophényle ou phtalimido] ou bien un halogénure d'acide, par exemple le chlorure d'acide.

Lorsque l'on met en œuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou dichlorométhane), un amide (par exemple diméthylformamide ou diméthylacétamide) ou une cétone (par exemple l'acétone), ou dans des mélanges de tels solvants [en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine)] ou dans un milieu hydroorganique en présence d'un agent alcalin de condensation tel que le bicarbonate de sodium, et l'on opère à une température comprise entre − 40 et + 40 °C.

Lorsque l'on met en œuvre un ester réactif de formule générale (XI) on opère généralement en présence d'une trialcoylamine (par exemple triéthylamine) dans un solvant organique tel que le diméthylformamide, à une température comprise entre 0 et 40 °C.

Les produits de formule générale (IX) peuvent être préparés à partir d'un produit de formule générale :

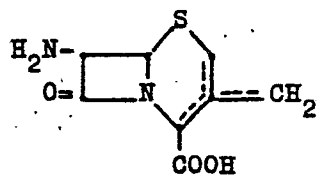

(XII)

[dans laquelle la position de la double liaison est définie comme pour le produit de formule générale (IX)] par application des méthodes décrites dans la littérature :

— lorsque $R_2$ est méthoxyméthyle : selon S. Seki Et coll., Tetrahedron Lett., 33, 2915 (1977) ;

— lorsque $R_2$ est t.butyle : selon R.J. Stedman, J. Med. Chem., 2, 444, (1966) ;

— lorsque $R_2$ est benzhydryle : selon la demande de brevet néerlandais 73 03263 ;

— lorsque $R_2$ est benzyle, nitrobenzyle ou p.méthoxybenzyle : selon R.R. Chauvette et coll., J. Org. Chem., 38 (17), 2994 (1973).

Les dérivés de la céphalosporine de formules générales (VII) et (IX) dans lesquelles $R_2$ représente un radical de formule générale (II), peuvent être obtenus par estérification de l'acide correspondant par toute méthode connue en soi pour préparer un ester à partir d'un acide sans toucher au reste de la molécule.

Généralement on fait réagir un sel alcalin ou un sel d'amine tertiaire d'un produit de formules générales :

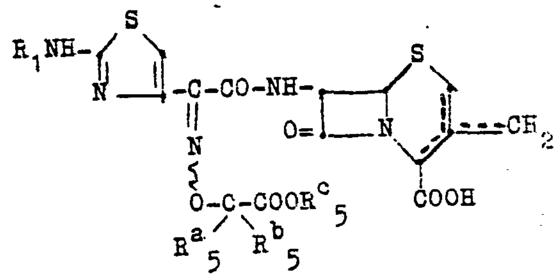

(XIII)

(dans laquelle les symboles sont définis comme précédemment) ou (XII) [dans lesquelles la position de la double liaison est définie comme pour les produits de formule générale (VII)] sur un halogénure de formule générale :

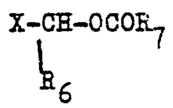

(XIV)

dans laquelle $R_6$ et $R_7$ sont définis comme précédemment et X représente un atome d'halogène, dans un solvant inerte tel que le diméthylformamide, à une température comprise entre 0 et 30 °C.

Les composés de formule générale (XIII) peuvent être préparés à partir des produits de formule générale (XII) par analogie avec la préparation des produits de formule générale (VII) à partir des produits de formule générale (IX).

Les halogénures de formule générale (XIV) peuvent être préparés selon la méthode décrite dans la demande de brevet allemand 23 50230.

Les produits de formule générale (X) peuvent être préparés par application de la méthode décrite dans les brevets belges 864 810, 865 298, 876 541 et 876 542.

Les nouveaux produits de formule générale (I) sont utiles comme intermédiaires pour la préparation de thiovinyl-3 céphalosporines qui sont décrites dans la demande EP 53961 et répondent à la formule générale :

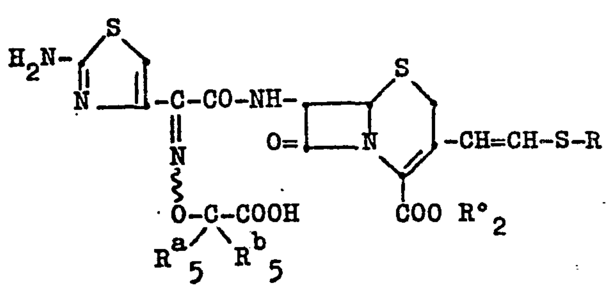

(XV)

dans laquelle

le symbole R est choisi parmi les significations suivantes :

1) alcoyle, L-amino-2 carboxy-2 éthyle, phényle

2) pyridyle-2, pyridyle-3 ou pyridyle-4 éventuellement N-oxydés

3) pyrimidinyle-2, pyridazinyle-3 substitué en position -6 (par un radical alcoyle, méthoxy, amino ou acylamino) et éventuellement N-oxydé ou tétrazolo [4,5-b] pyridazinyle-6

4) dioxo-5,6 tétrahydro-1,4,5,6 triazine-1,2,4 yle-3 substitué en position -4, triazol-1,3,4 yle-5 ou alcoyloxycarbonyl-2 triazol-1,3,4 yle-5 substitués en position -1

a) par un radical alcoyle contenant 1 à 4 atomes de carbone non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle, dihydroxy-2,3 propyle, dihydroxy-1,3 propyle-2, formyl-2 hydroxy-2 éthyle, formyloxy-3 hydroxy-2 propyle, bis-formyloxy-2,3 propyle ou bis-formyloxy-1,3 propyle-2,

c) par un radical alcoyle contenant 2 à 4 atomes de carbone, substitué par hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales :

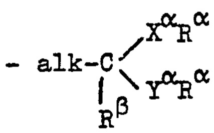 (XVIa) ou 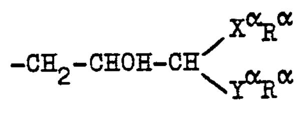 (XVIb)

ou 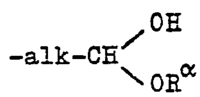 (XVIc)

dans lesquelles alk est un radical alcoylène contenant 1 à 4 atomes de carbone, $X^\alpha$ et $Y^\alpha$ sont identiques et représentent des atomes d'oxygène ou de soufre, et $R^\alpha$ représente un radical alcoyle, ou bien $X^\alpha$ et $Y^\alpha$ sont identiques ou différents et représentent des atomes d'oxygène ou de soufre, et les radicaux $R^\alpha$ forment ensemble un radical alcoylène contenant 2 ou 3 atomes de carbone, et $R^\beta$ représente un atome d'hydrogène ou un radical alcoyle contenant 1 à 3 atomes de carbone,

e) par un radical alcoyle contenant 2 à 5 atomes de carbone substitué par un radical alcoyloxyimino ou hydroxyimino,

5) dialcoyl-1,4 dioxo-5,6 tétrahydro-1,4,5,6 triazine-1,2,4 yle-3, alcoyl-1 dioxo-5,6 tétrahydro-1,4,5,6 triazine-1,2,4 yle-3, alcoxyl-2 dioxo-5,6 tétrahydro-1,2,5,6 triazine-1,2,4 yle-3

6) triazol-1,3,4 yle-5, triazol-1,2,3 yle-5 ou alcoyl-1 triazol-1,2,4 yle-5 non substitué ou substitué en position -3 par alcoyloxycarbonyle

7) a) thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) thiadiazol-1,2,4 yle-5 substitué par un radical alcoyle ou alcoyloxy,

8) a) oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle

b) oxazolyle-2 ou alcoyl-4 oxazolyle-2,

9) tétrazolyle-5 non substitué ou substitué en position -1 par

a) un radical alcoyle contenant 1 à 4 atomes de carbone non substitué ou substitué par alcoyloxy, sulfo, carboxy, formyle ou sulfamoyle,

b) un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) un radical alcoyle contenant 2 à 5 atomes de carbone substitué par hydroxyimino ou alcoyloxyimino,

d) un radical phényle, dihydroxy-2,3 propyle, dihydroxy-1,3 propyle-2, formyl-2 hydroxy-2 éthyle, formyloxy-3 hydroxy-2 propyle, bis-formyloxy-2,3 propyle ou bis-formyloxy-1,3 propyle-2,

e) un radical de formule générale (XVIa) pour lequel $R^\beta$ représente un atome d'hydrogène, ou un radical de formule générale (XVIb),

10) dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, triazol-1,2,4 yle-5 ou alcoyloxycarbonyl-3 triazol-1,2,4-yle-5 substitués en position -1 par

a) un radical alcoyle lui-même substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle, alcoyloxycarbonyle ou thiazolidinyle-2 ou par

b) un radical tel que défini précédemment en 4 b), c), d) et e),

11) dioxo-5,6 hydroxyalcoylcarbamoylalcoyl-4 tétrahydro-1,4,5,6 triazine-1,2,4 yle-3 dont la partie hydroxyalcoyle contient 2 à 4 atomes de carbone, ou bien

12) alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle ou par un radical de formule générale (XVIa) dans laquelle $R^{\beta}$ représente un atome d'hydrogène ; et

le symbole $R^{o}_{2}$ représente un atome d'hydrogène ou un radical de formule générale (II).

Les produits de formule générale (XV) peuvent être obtenus à partir des produits de formule générale (I) en opérant comme suit :

On prépare un produit de formule générale :

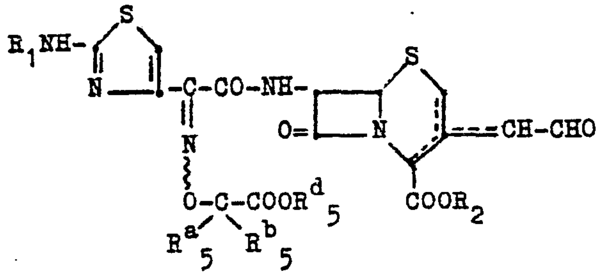

(XVII)

[dans laquelle $R^{d}_{5}$ est défini comme $R^{c}_{5}$ ou représente un atome d'hydrogène, et les autres symboles sont définis comme ci-dessus, et qui se présente sous forme bicyclooctène-2 ou -3 ou oxoéthylidène-3 bicyclooctane] par hydrolyse de l'énamine de formule générale (I) selon l'invention ou du mélange de ses isomères, puis éventuellement élimination du radical protecteur $R^{c}_{5}$.

De préférence on hydrolyse une énamine de formule générale (I) dans laquelle $R_3$ et $R_4$ représentent un radical méthyle.

On opère généralement dans un acide organique (par exemple acide formique, acide acétique) ou minéral (par exemple acide chlorhydrique, acide sulfurique) en présence ou non d'un solvant, en milieu aqueux ou organique, à une température comprise entre − 20 °C et la température de reflux du mélange réactionnel, puis on traite éventuellement par une base minérale (bicarbonate alcalin) ou organique (amine tertiaire ou pyridine).

Lorsque l'on opère en milieu organique, l'hydrolyse est réalisée par addition d'eau au mélange réactionnel.

Lorsque l'on opère en présence d'un solvant, il n'est pas nécessaire que le solvant soit miscible à la phase aqueuse acide. Le contact est alors réalisé par agitation vive.

Parmi les solvants utilisables peuvent être cités les solvants chlorés, l'acétate d'éthyle, le tétrahydrofuranne, l'acétonitrile, le diméthylformamide, les alcools.

Il n'est pas absolument nécessaire d'avoir purifié l'intermédiaire de formule générale (I) pour mettre en œuvre cette réaction.

Lorsque l'on veut préparer un produit de formule générale (XVII) dans laquelle $R^{d}_{5}$ représente un atome d'hydrogène, il est nécessaire d'utiliser une énamine de formule générale (I) dans laquelle les radicaux protecteurs des fonctions acides ($R^{c}_{5}$ et $R_2$) soient différents et éliminables sélectivement.

L'élimination du radical protecteur $R^{c}_{5}$ s'effectue dans des conditions qui seront décrites ci-après.

On peut oxyder le produit de formule générale (XVII) en produit de formule générale :

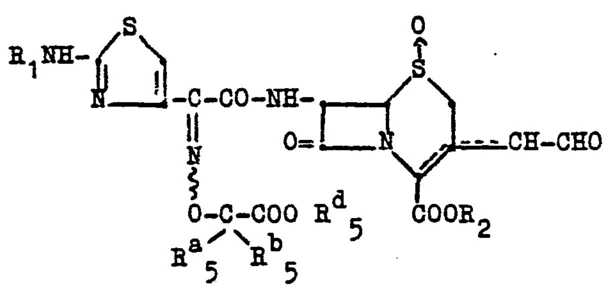

(XVIII)

(dans laquelle les symboles ont la définition correspondante, et qui se présentent sous forme bicyclooctène-2 ou oxoéthylidène-3 bicyclooctane) par application de la méthode décrite dans la demande de brevet allemande 26 37176.

On prépare ensuite un produit de formule générale :

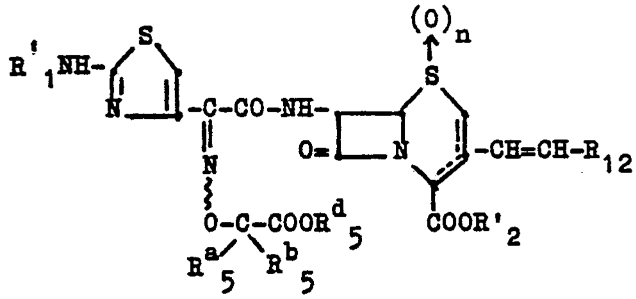

(XIX)

[dans laquelle

R'$_1$ représente l'hydrogène ou un radical $R_1$ tel que défini précédemment ;

R'$_2$ représente l'hydrogène ou un radical $R_2$ tel que défini précédemment ;

$R^a_5$, $R^b_5$ et $R^d_5$ sont définis comme précédemment (étant entendu que lorsque n = 0 le produit se présente sous forme bicyclooctène-2 ou -3 et lorsque n = 1 le produit se présente sous forme bicyclooctène-2) ; le substituant sur l'atome de carbone en position -3 du bicyclooctène présente la stéréoisomérie E ou Z et le symbole $R_{12}$ représente un radical de formule générale :

$$R'_{12}—SO_2O— \text{ (XX)} \quad \text{ou} \quad R''_{12}—COO— \text{ (XXI)}$$

(dans lesquelles $R'_{12}$ représente un radical alcoyle, trifluorométhyle, trichlorométhyle ou un radical phényle non substitué ou substitué par un atome d'halogène ou par un radical alcoyle ou nitro et $R''_{12}$ est défini comme $R_{12}$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle), ou un atome d'halogène choisi parmi le chlore, le brome et l'iode] par action d'une forme activée d'un acide $R'_{12}—SO_3H$ ou $R''_{12}—COOH$, de formules :

$$(R'_{12}SO_2)_2O \text{ (XXII)} \quad R'_{12}SO_2\text{ Hal (XXIII)}$$
$$(R''_{12}CO)_2O \text{ (XXIV)} \quad R''_{12}CO\text{ Hal (XXV)}$$

(Hal représentant un atome d'halogène)

ou par action d'un réactif d'halogénation, sur un produit de formule générale (XVII) ou (XVIII) ou sur un mélange de ses isomères, suivie éventuellement de la réduction du sulfoxyde obtenu et, le cas échéant, de l'élimination des groupements protecteurs de la fonction amine ($R_1$) et/ou éventuellement des fonctions acides ($R_2$ et/ou $R^d_5$) lorsque l'on veut obtenir un produit de formule générale (XIX) pour lequel les fonctions amine et/ou acide sont libres.

On opère généralement en présence d'une base tertiaire de formule générale :

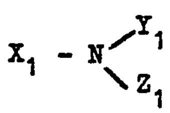

(XXVI)

dans laquelle $X_1$, $Y_1$ et $Z_1$ représentent des radicaux alcoyle ou phényle, ou éventuellement deux d'entre eux forment un cycle avec l'atome d'azote auquel ils sont rattachés (par exemple en présence de triéthylamine ou de NN-diméthylaniline), dans un solvant organique chloré (par exemple dichlorométhane), dans un ester (par exemple acétate d'éthyle), dans un éther (par exemple dioxanne, tétrahydrofuranne), dans un amide (par exemple diméthylacétamide, diméthylformamide), dans l'acétonitrile ou la N-méthylpyrrolidone ou dans un mélange de tels solvants ou directement dans un solvant basique comme la pyridine, ou bien lorsque $R_{12}$ est autre qu'un atome d'halogène, on peut opérer en milieu hydroorganique en présence d'un agent alcalin de condensation (par exemple bicarbonate alcalin, soude ou potasse), à une température comprise entre − 78 °C et la température de reflux du mélange réactionnel.

Eventuellement la réaction est mise en œuvre sous azote.

Il n'est pas absolument nécessaire d'avoir purifié l'intermédiaire de formules générales (XVII) ou (XVIII) pour mettre en œuvre cette réaction.

Lorsque l'on veut préparer un produit de formule générale (XIX) dans laquelle $R_{12}$ est un atome d'halogène, on effectue la réaction à partir d'un produit de formule générale (XVII) ou (XVIII) dans laquelle $R^d_5$ est un radical protecteur $R^c_5$. Les agents d'halogénation peuvent être choisis parmi des dérivés halogénés du phosphore, notamment

— les composés d'addition d'halogène et de triarylphosphite, ou bien

— le trichlorure de phosphore, l'oxychlorure de phosphore, le pentachlorure de phosphore, le dichlorotriphénylphosphorane ou le catéchyltrichlorophosphorane lorsque $R_{12}$ est un atome de chlore, ou

— le tribromure de phosphore, l'oxybromure de phosphore, le pentabromure de phosphore ou le catéchyltribromophosphorane lorsque $R_{12}$ est un atome de brome.

Lorsque l'on utilise le trichlorure (ou le tribromure) de phosphore, on fait agir ce réactif sur un produit de formule générale (XVII).

Le catéchyltrichloro (ou tribromo) phosphorane, qui peut être préparé in situ, peut être obtenu selon la méthode décrite par H. Gross et U. Karsch, J. Prakt. Chem., 29, 315 (1965).

Les composés d'addition d'halogène et de triarylphosphite, qui peuvent être formés in situ, sont décrits par H. N. Rydon et B. L. Tonge, J. Chem. Soc., 3 043 (1956), par J. Michalski et coll., J. Org. Chem., 45, 3 122 (1980) ou dans le brevet belge 881 424 et peuvent être préparés selon les méthodes mentionnées dans ces documents.

La préparation des dérivés halogénés de formule générale (XIX) s'effectue en milieu anhydre.

Lorsque l'on veut préparer un produit de formule générale (XIX), dans laquelle $R_{12}$ est un atome de chlore ou de brome, selon les conditions opératoires on peut isoler l'intermédiaire dihalogéné de formule générale :

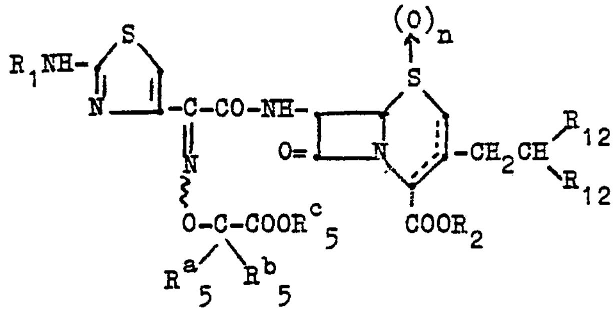

(XIXa)

[dans laquelle $R_1$, $R_2$, $R^a_5$, $R^b_5$ et $R^c_5$ sont définis comme dans la formule générale (XVII) et $R_{12}$ est défini comme ci-dessus, et qui se présente sous la forme bicyclooctène-2 (ou -3) lorsque n = 0 ou sous la forme bicyclooctène-2 lorsque n = 1] qui est ensuite déhydrohalogéné.

Lorsque l'on veut isoler l'intermédiaire dihalogéné, on opère par action d'un agent d'halogénation, dans un solvant organique tel qu'un solvant chloré (dichlorométhane, chloroforme, tétrachlorure de carbone, dichloro-1,2 éthane par exemple), un éther (éther éthylique, oxyde de propylène, tétrahydrofuranne, dioxanne par exemple), un amide (diméthylacétamide, diméthylpropionamide, diméthylformamide, N-acétylmorpholine, N-acétylpipéridine, N-méthylpyrrolidone par exemple) ou un mélange de tels solvants, à une température un peu moins élevée que pour préparer le dérivé halogénovinyle correspondant, c'est-à-dire comprise entre − 78 et 30 °C. Il est également possible d'opérer en présence d'une base telle que la pyridine dans l'un des solvants ci-dessus, à une température comprise entre − 78 et 0 °C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin ou alcalino-terreux), en milieu organique ou hydroorganique dans les solvants cités précédemment, à une température comprise entre − 20 °C et la température de reflux du mélange réactionnel.

Il n'est pas absolument nécessaire d'avoir purifié l'intermédiaire dihalogéné pour procéder à sa déhydrohalogénation.

La réduction du S-oxyde peut être effectuée dans les conditions décrites dans la demande de brevet allemand 2 637 176.

Le cas échéant, l'élimination des radicaux protecteurs de la fonction amine ($R_1$) et des fonctions acides ($R^c_3$ et/ou $R_2$) peut être effectuée simultanément ou successivement.

A titre d'exemple :

1. L'élimination des groupements protecteurs d'amines s'effectue

— lorsqu'il s'agit d'un radical t.butoxycarbonyle, trityle, p.méthoxybenzyloxycarbonyle ou formyle : par traitement en milieu acide. De préférence on utilise l'acide trifluoroacétique en opérant à une température comprise entre 0 et 20 °C, ou bien on utilise l'acide formique, phosphorique ou polyphosphorique purs ou en présence d'eau à température comprise entre 20 et 60 °C, ou encore l'acide paratoluènesulfonique ou méthanesulfonique dans l'acétone à la température de reflux du mélange réactionnel. Dans ces conditions le produit de formule générale (XIX) peut être obtenu sous forme de trifluoroacétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique

— lorsqu'il s'agit d'un radical trichloro-2,2,2,2 éthoxycarbonyle ou p.-nitrobenzyloxycarbonyle : par réduction (notamment traitement par le zinc dans l'acide acétique)

— lorsqu'il s'agit d'un radical chloracétyle ou trichloracétyle : par application de la méthode décrite dans le brevet français publié sous le n° 2 243 199

— lorsqu'il s'agit d'un radical benzyle, dibenzyle ou benzyloxycarbonyle : par hydrogénation catalytique

— lorsqu'il s'agit d'un radical trifluoroacétyle : par traitement en milieu basique.

2. L'élimination des groupements protecteurs de radical carboxy s'effectue

— lorsqu'il s'agit d'un groupement t.-butyle, p.-méthoxybenzyle ou benzhydryle : par traitement en milieu acide, dans les conditions décrites ci-dessus pour l'élimination du radical trityle protecteur d'amino. Dans le cas du radical benzhydryle, on peut opérer en présence d'anisole

— lorsqu'il s'agit d'un groupement méthoxyméthyle : par traitement en milieu acide dilué

— lorsqu'il s'agit d'un groupement nitrobenzyle : par réduction (notamment traitement par le zinc dans l'acide acétique ou hydrogénolyse).

I. Les thiovinyl-3 céphalosporines de formule générale (XV) dans laquelle R ne contient pas de substituant de formule générale (XVIc) peuvent être préparées par action d'un thiol (ou d'un de ses sels alcalins ou alcalino-terreux) de formule générale :

R—SH (XXVII)

dans laquelle R, qui est défini comme précédemment à l'exception de contenir un substituant de formule générale (XVIc), est éventuellement protégé, sur un dérivé de la céphalosporine (ou un mélange des isomères) de formule générale (XIX) dans laquelle les symboles sont définis comme précédemment, suivie de la réduction du sulfoxyde obtenu (lorsque n = 1), puis éventuellement de l'élimination des radicaux protecteurs.

Lorsque l'on veut obtenir un produit de formule générale (XV) dans laquelle R contient un radical formyle ou acylalcoyle, on met en œuvre un thiol de formule générale (XXVII) dans laquelle ce radical est protégé à l'état d'acétal [tel que défini par les formules générales (XVIa) et (XVIb)].

Il est entendu que, lorsque le radical R du produit de formule générale (XXVII) contient un groupement susceptible d'interférer avec la réaction, il est préférable de protéger ce groupement par toute méthode connue en soi et qui n'altère pas le reste de la molécule.

S'il s'agit d'un groupement amino ou alcoylamino, la protection s'effectue par un radical tel que $R_1$ défini précédemment.

S'il s'agit d'un groupement carboxy la protection s'effectue par un radical tel que méthoxyméthyle, t.butyle, benzhydryle, p.nitrobenzyle ou p.méthoxybenzyle.

S'il s'agit de groupements hydroxy la protection s'effectue par un radical tel que trityle, tétrahydropyrannyle, méthoxy-2 propyle-2 ou bien, lorsqu'il s'agit des groupements dihydroxy-2,3 propyle ou dihydroxy-1,3 propyle-2, à l'état d'acétal cyclique sous forme de radicaux diméthyl-2,2 dioxolannyl-4 méthyle ou diméthyl-2,2 dioxannyle-5.

Par ailleurs il est entendu que, lorsque le radical R du produit de formule générale (XXVII) comporte un radical hydroxy, sulfo, sulfinyle ou sulfonyle, il est préférable de mettre en œuvre un produit de formule générale (XIX) dans laquelle n = 0.

La réaction des produits de formules générales (XXVII) et (XIX) s'effectue généralement en présence d'une base telle qu'une pyridine ou une base organique tertiaire de formule générale (XXVI). On utilise par exemple la diisopropyléthylamine ou la diéthylphénylamine.

Lorsque l'on utilise un sel du thiol de formule générale (XXVII) il n'est pas nécessaire d'opérer en présence d'une base organique telle que définie ci-dessus.

On opère avantageusement dans un solvant organique tel que le diméthylformamide, le tétrahydrofuranne, le méthanol, l'éthanol ou l'acétonitrile ou un mélange de tels solvants.

Il est également possible d'opérer en présence de bicarbonate alcalin dans un solvant tel que cité ci-dessus, éventuellement en présence d'eau. On opère à une température comprise entre − 20 °C et la température de reflux du mélange réactionnel, la température choisie étant variable selon le thiol employé. De même, selon le thiol employé, le temps de réaction peut varier de 5 minutes à 48 heures. Eventuellement on opère sous azote.

De préférence, lorsque l'on veut utiliser un bicyclooctène-3 de formule générale (XIX), on met en œuvre un tel produit pour lequel $R'_2$ est autre que l'hydrogène et $R^d_5$ est défini comme précédemment.

La réduction de l'oxyde et l'élimination des groupements protecteurs d'amine ou d'acide s'effectuent selon les méthodes décrites précédemment.

L'élimination des radicaux protecteurs de groupements hydroxy s'effectue :

— par acidolyse, par exemple par l'acide trifluoroacétique, l'acide formique aqueux ou non ou l'acide paratoluènesulfonique lorsqu'il s'agit du radical trityle, tétrahydropyrannyle, diméthyl-2,2 dioxolannyl-4 méthyle ou diméthyl-2,2 dioxannyle-5 [Lorsque l'on utilise l'acide formique aqueux ou non, la libération des radicaux hydroxy protégés peut conduire au moins partiellement aux esters formiques correspondants, qui peuvent être séparés le cas échéant par chromatographie],

— selon la méthode décrite dans le brevet belge 875 379 lorsqu'il s'agit du radical méthoxy-2 propyle-2.

L'élimination des radicaux protecteurs dans les groupements de formule générale (XVIa) ou (XVIb) (lorsque l'on veut obtenir un produit de formule générale (XV) dans laquelle R contient un radical formule ou acylalcoyle) s'effectue

— en présence d'un acide sulfonique (acide méthanesulfonique ou acide p.toluènesulfonique par exemple) dans un solvant organique (acétonitrile ou acétone par exemple), éventuellement en présence d'eau et éventuellement en présence d'un réactif acétalisable tel que l'acétone, l'acide glyoxylique, le benzaldéhyde ou l'acide pyruvique, à une température comprise entre 20 °C et la température de reflux du mélange réactionnel

— ou bien, lorsque le radical R est un radical dioxo-5,6 tétrahydro-1,4,5,6 triazine-1,2,4 yle-3, par action d'acide formique pur ou aqueux (contenant de préférence moins de 10 % d'eau), soit en présence ou non de silice, soit par transacétalisation en présence d'un réactif acétalisable tel que défini ci-dessus.

Les thiols de formule générale (XXVII) (qui peuvent être mis en œuvre sous leur forme tautomère) peuvent être préparés par application de l'une des méthodes suivantes selon la signification du radical R :

— lorsque R est un radical pyridyle-3 : selon la méthode décrite par H. M. Wuest et E. H. Sakal, J. Am. Chem. Soc., 73, 1210 (1951),

— lorsque R est un radical oxyde-1 pyridyle-3 : selon la méthode décrite par B. Blank et coll., J. Med. Chem. 17, 1065 (1974),

— lorsque R est un radical oxyde-1 pyridyle-4 : selon la méthode décrite par R. A. Y. Jones et coll., J. Chem. Soc. 2937 (1960),

— lorsque R est un radical pyridazinyle-3 substitué par alcoyle ou méthoxy et éventuellement N-oxydé : selon la méthode décrite dans le brevet belge 787 635,

— lorsque R est un radical pyridazinyle-3 substitué par amino et éventuellement N-oxydé : selon la méthode décrite dans le brevet belge 579 291,

— lorsque R est un radical pyridazinyle-3 substitué par acylamino et éventuellement N-oxydé : par application des méthodes décrites par M. Kumagai et M. Bando, Nippon Kagaku Zasshi, 84, 995 (1963) et par T. Horie et R. Ueda, Chem. Pharm. Bull., 11, 114 (1963),

— lorsque R est un radical tétrazolo [4,5-b] pyridazinyle-6 : selon la méthode décrite dans le brevet belge 804 251,

— lorsque R est un radical dioxo-5,6 tétrahydro-1,4,5,6 triazine-1,2,4 yle-3 substitué en position -4 ou alcoyloxycarbonyl-2 triazol-1,3,4 yle-5 substitué en position -1 par un radical $R^γ$ choisi parmi :

a) un radical allyle, alcoyle (1 à 4 atomes de carbone, lui-même éventuellement substitué par un radical alcoyloxy, alcoylthio, phényle, carbamoyle, alcoylcarbamoyle, dialcoylcarbamoyle, acyle, alcoyloxycarbonyle ou thiazolidinyle-2)

b) un radical dihydroxy-2,3 propyle ou dihydroxy-1,3 propyle-2 (éventuellement protégé sous forme d'acétal cyclique)

c) un radical alcoyle [2 à 4 atomes de carbone, lui-même substitué par hydroxy, carbamoyloxy, dialcoyloamino, alcoylsulfinyle, alcoylsulfonyle, alcoylsulfonylamino, sulfamoylamino, acylamino (éventuellement substitué), alcoyloxycarbonylamino, uréido, alcoyluréido, dialcoyluréido],

d) un radical de formule générale (XVIa) ou (XVIb),

e) un radical hydroxyiminoalcoyle ou alcoyloxyiminoalcoyle,

ou lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 substitué en position -1 par un radical $R^γ$ tel que défini ci-dessus (à l'exception de représenter un radical alcoyle non substitué) ou par un radical hydroxyalcoylcarbamoylalcoyle dont la portion hydroxyalcoyle contient 2 à 4 atomes de carbone, ou encore

lorsque R est un radical dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone :

en faisant agir un oxalate d'alcoyle ou un halogénure de monooxalate d'alcoyle sur une thiosemicarbazide de formules générales :

$H_2N-CS-NH-NH-R^Y$ (XXVIIIa)

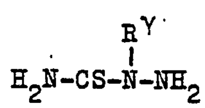

(XXVIIIb)

ou

$R^{Y1}-NH-CS-NH-NH_2$ (XXVIIIc)

(dans lesquelles $R^γ$ est défini comme ci-dessus et $R^{γ1}$ représente un radical hydroxyalcoylcarbamoylalcoyle ou un radical $R^γ$).

On effectue la réaction en présence d'un alcoolate alcalin, par exemple l'éthylate ou le méthylate de sodium ou le t.-butylate de potassium, par application de la méthode décrite par M. Pesson et M. Antoine, Bull. Soc. Chim. France 1590 (1970).

Il n'est pas absolument nécessaire de purifier le produit obtenu (ni de libérer les radicaux protégés pour le mettre en œuvre pour la préparation des produits de formule générale (XV).

Les thiosemicarbazides de formules générales (XXVIIIa) à (XXVIIIc) peuvent être préparées selon l'une des méthodes décrites par K. A. Jensen et coll., Acta Chem. Scand., 22, 1 (1968), ou par application

de la méthode décrite par Y. Kazakov et J. Y. Potovskii, Doklady Acad. Nauk. SSSR, 134, 824 (1960), étant entendu que, lorsque $R^γ$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxycarbonyle, qui peut être éliminé par hydrolyse acide.

— Lorsque R est un radical triazol-1,3,4 yle-5 substitué en position -1 par un radical alcoyle, allyle ou alcoyloxyalcoyle, par un radical alcoyle (1 à 4 atomes de carbone) lui-même substitué comme défini ci-dessus en a) à l'exception d'un radical thiazolidinyle-2, par un radical tel que défini ci-dessus en c), ou par un radical alcoyloxyiminoalcoyle : par application de l'une des méthodes décrites par M. Pesson et M. Antoine, Bull. Soc. Chim. France 1590 (1970) ;

— Lorsque R est un radical triazol-1,3,4 yle-5 substitué en position-1 par thiazolidinyl-2 alcoyle ou hydroxyiminoalcoyle ; par action respectivement de cystéamine ou d'hydroxylamine sur un dialcoyloxyalcoyl-1 mercapto-5 triazole-1,3,4 qui peut être obtenu par application de la méthode décrite par M. Kanaoka, J. Pharm. Soc. Japan, 75, 1149 (1955), à partir d'une dialcoyloxyalcoyl-4 thiosemicarbazide.

— Lorsque R est un radical triazol-1,3,4 yle-5 substitué en position -1 par dihydroxy-2,3 propyle ou dihydroxy-1,3 propyle-2 (éventuellement protégés sous forme d'acétal cyclique), ou représente un radical de formule générale (XVIa) ou (XVIb) : par application de la méthode décrite par M. Kanaoka, J. Pharm. Soc. Japan, 75, 1149 (1955).

— Lorsque R est un radical dioxo-5,6 tétrahydro-1,4,5,6 triazine-1,2,4 yle-3 substitué en position -4 ou un radical alcoyloxycarbonyl-2 triazol-1,3,4 yle-5 ou triazol-1,3,4 yle-5 substitués en position -1 par acyloxyalcoyle (éventuellement substitué) : par acylation respectivement de la dioxo-5,6 hydroxyalcoyl-4 mercapto-3 tétrahydro-1,4,5,6 triazine-1,2,4, de l'alcoyloxycarbonyl-2 hydroxyalcoxyl-1 mercapto-5 triazole-1,3,4 ou de l'hydroxyalcoyl-1 mercapto-5 triazole-1,3,4 dont le radical mercapto a été préalablement protégé [par exemple selon C. G. Kruse et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position -4 ou alcoyloxycarbonyl-2 triazol-1,3,4 yle-5 ou triazol-1,3,4 yle-5 substitués en position -1 par aminoalcoyle ou alcoylaminoalcoyle : par libération de la fonction amine du produit correspondant, protégée par exemple par un groupement t.butoxycarbonyle.

— Lorsque R est un radical dioxo-5,6 tétrahydro-1,4,5,6 triazine-1,2,4 yle-3 substitué en position -4, alcoyloxycarbonyl-2 triazol-1,3,4 yle-5 ou triazol-1,3,4 yle-5 substitués en position -1 par sulfoaminoalcoyle : à partir du produit correspondant substitué par un radical t.butoxycarbonylaminoalcoyle, par analogie avec la méthode décrite dans le brevet belge 847 237.

— Lorsque R est un radical dialcoyl-1,4 dioxo-5,6 tétrahydro-1,4,5,6 triazine-1,2,4 yle-3 ou alcoyl-1 dioxo-5,6 tétrahydro-1,4,5,6 triazine-1,2,4 yle-3 ; selon la méthode décrite dans le brevet belge 830 455.

— Lorsque R est un radical alcoyl-2 dioxo-5,6 tétrahydro-1,2,5,6 triazine-1,2,4 yle-3 ou alcoyl-1 alcoyloxycarbonyl-3 triazol-1,2,4 yle-5 : selon la méthode décrite par M. Pesson et M. Antoine, C.R. Acad. Sci., Ser C, 267, 25, 1726 (1968).

— Lorsque R est un radical triazol-1,2,3 yle-5 : selon la méthode décrite dans la demande de brevet français 2 215 942.

— Lorsque R est un radical triazol-1,3,4 yle-5 : selon la méthode décrite par M. Kanaoka, J. Pharm. Soc. Jap. 75, 1149 (1955).

— Lorsque R est un radical thiadiazol-1,3,4 yle-5 éventuellement substitué par alcoyle, alcoyloxy, alcoylthio, alcoylsulfonyle, amino, alcoylamino, dialcoylamino ou acylamino : selon les méthodes décrites dans le brevet belge 830 821.

— Lorsque R est un radical thiadiazol-1,3,4 yle-5 substitué par hydroxyalcoyle, aminoalcoyle, alcoylaminoalcoyle ou dialcoylaminoalcoyle : selon la méthode décrite dans la demande de brevet allemand 2 446 254.

— Lorsque R est un radical thiadiazol-1,3,4 yle-5 substitué par un radical carboxyalcoyle : par application de la méthode décrite dans la demande de brevet allemand 1 953 861.

— Lorsque R est un radical thiadiazol-1,3,4 yle-5 substitué par un radical trifluorométhyle : selon la méthode décrite dans la demande de brevet allemand 2 162 575.

— Lorsque R est un radical thiadiazol-1,3,4 yle-5 substitué par un radical carboxy : selon la méthode décrite dans la demande de brevet japonais 77 48666.

— Lorsque R est un radical thiadiazol-1,3,4 yle-5 substitué par un radical acylaminoalcoyle : selon la méthode décrite dans la demande de brevet japonais 76 80857.

— Lorsque R est un radical thiadiazol-1,3,4 yle-5 substitué par un radical hydroxyalcoylthio : par application de la méthode décrite par G. Nannini, Arz. Forsch. 27 (2), 343 (1977).

— Lorsque R est un radical thiadiazol-1,2,4 yle-5 substitué par alcoyle ou alcoyloxy : selon la méthode décrite dans la demande de brevet allemand 2 806 226 ou selon Chem. Ber. 90, 184 (1957).

— Lorsque R est un radical oxadiazol-1,3,4 yle-5 tel que défini précédemment en 8a) : par application de la méthode décrite par E. Hoggarth, J. Chem. Soc. 4811 (1952).

— Lorsque R est un radical oxazolyle-2 ou alcoyl-4 oxazolyle-2 : par application de la méthode décrite précédemment par C. Bradsher, J. Org. Chem. 32, 2079 (1967).

— Lorsque R est un radical tétrazolyle-5 éventuellement substitué en position -1 par alcoyle, hydroxyalcoyle ou phényle : selon les méthodes décrites dans le brevet belge 830 821.

— Lorsque R est un radical tétrazolyle-5 substitué en position -1 par alcoyloxyalcoyle : par addition d'azoture de sodium sur un isothiocyanatoalcoyloxyalcoyle en opérant dans un solvant organique tel que l'éthanol, à la température de reflux du mélange réactionnel.

L'isothiocyanatoalcoyloxyalcoyle peut être obtenu par application de la méthode décrite par E. Schmidt et coll., Chem. Ber. 73, 286 (1940).

— Lorsque R est un radical tétrazolyle-5 substitué en position -1 par un radical carboxyalcoyle : selon la méthode décrite dans le brevet belge 858 112.

— Lorsque R est un radical tétrazolyle-5 substitué en position -1 par un radical sulfoalcoyle : selon la méthode décrite dans le brevet belge 856 498 ou décrite par D. A. Berges et coll., J. Het. Chem. 15, 981 (1978).

— Lorsque R est un radical tétrazolyle-5 substitué en position -1 par un radical aminoalcoyle, alcoylaminoalcoyle, dialcoylaminoalcoyle : par application de la méthode décrite dans la demande de brevet allemand 2 738 711.

— Lorsque R est un radical tétrazolyle-5 substitué en position -1 par un radical sulfamoylalcoyle, sulfamoylaminoalcoyle ou sulfoaminoalcoyle : selon la méthode décrite dans le brevet belge 856 636.

— Lorsque R est un radical tétrazolyle-5 substitué par un radical acylaminoalcoyle ou thiadiazol-1,3,4 yle-5 substitué par hydroxy : selon la méthode décrite dans le brevet US 4 117 123.

— Lorsque R est un radical tétrazolyle-5 substitué en position -1 par un radical uréidoalcoyle, alcoyluréidoalcoyle ou dialcoyluréidoalcoyle : à partir du produit correspondant substitué par aminoalcoyle (dont le radical mercapto a été préalablement protégé), par traitement par un isocyanate alcalin, par un isocyanate d'alcoyle ou par un halogénure de dialcoylcarbamoyle, puis libération du groupement mercapto dans les conditions décrites dans le brevet belge 847 237.

— Lorsque R est un radical tétrazolyle-5 substitué en position -1 par un radical carboxyalcoylaminoalcoyle : selon la méthode décrite dans la demande de brevet allemand 2 715 597.

— Lorsque R est un radical tétrazolyle-5 substitué en position -1 par un radical dihydroxy-2,3 propyle : selon la méthode décrite dans le brevet US 4 064 242.

— Lorsque R est un radical tétrazolyle-5 substitué en position -1 par un radical dihydroxy-1,3 propyle-2 : par addition d'azoture de sodium sur un isothiocyanate de diméthyl-2,2 dioxolanne-1,3 yle-5 (suivie éventuellement de la libération des groupements hydroxy).

— Lorsque R est un radical tétrazolyle-5 substitué en position -1 par un radical de formule générale (XVIa) ou (XVIb) tels que définis précédemment en 9e) ou un radical défini précédemment en 9c) : par action d'azoture de sodium sur l'isothiocyanate correspondant, par analogie avec la méthode décrite par R. E. Orth, J. Pharm. Sci. 52 (9), 909 (1963), étant entendu que dans le cas où R contient un substituant hydroxy ou hydroxyiminoalcoyle, l'alcool ou l'oxime sont éventuellement protégés par exemple par un groupement tétrahydropyrannyle.

— Lorsque R est un radical triazol-1,2,4 yle-5 substitué en position -1 par un radical allyle ou alcoyloxyalcoyle, par un radical alcoyle (1 à 4 atomes de carbone) lui-même substitué comme défini ci-dessus en 10a) à l'exception d'un radical thiazolidinyle-2, par un radical hydroxyalcoylcarbamoylalcoyle dont la partie hydroxyalcoyle contient 2 à 4 atomes de carbone, par un radical tel que défini ci-dessus en 4c), ou par un radical alcoyloxyiminoalcoyle : par analogie avec les méthodes décrites par M. Pesson et M. Antoine, Bull. Soc. Chim. France 1599 (1970) ou C.R. Acad. Sci., Ser C, 267, (25), 1726 (1968).

— Lorsque R est un radical triazol-1,2,4 yle-5 substitué en position -1 par thiazolidinyl-2 alcoyle ou par hydroxyiminoalcoyle : par action respectivement de cystéamine ou d'hydroxylamine sur un dialcoyloxyalcoyl-1 mercapto-5 triazole-1,2,4 qui peut être obtenu par analogie avec la méthode décrite par M. Kanaoka, J. Pharm. Soc. Japan, 75, 1149 (1955), à partir d'une dialcoyloxyalcoyl-4 thiosemicarbazide.

— Lorsque R est un radical triazol-1,2,4 yle-5 substitué en position -1 par dihydroxy-2,3 propyle ou dihydroxy-1,3 propyle-2 (éventuellement protégés sous forme d'acétal cyclique), ou par un radical de formule générale (XVIa) ou (XVIb) : par analogie avec la méthode décrite par M. Kanaoka, J. Pharm. Soc. Japan, 75, 1149 (1955).

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou un radical alcoyloxycarbonyl-3 triazol-1,2,4 yle-5 ou triazol-1,2,4 yle-5 substitués en position -1, par acyloxyalcoyle (éventuellement substitué) : par acylation respectivement de la dioxo-5,6 hydroxyalcoyl-1 mercapto-3 tétrahydro-1,4,5,6 triazine-1,2,4, de la dioxo-5,6 hydroxyalcoyl-2 mercapto-3 tétrahydro-1,2,5,6 triazine-1,2,4, de l'alcoyloxycarbonyl-3 hydroxyalcoyl-1 mercapto-5 triazole-1,2,4 ou de l'hydroxyalcoyl-1 mercapto-5 triazole-1,2,4 dont le radical mercapto a été préalablement protégé [par exemple selon C. G. Kruse et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 ou triazol-1,2,4 yle-5 substitués en position -1, par aminoalcoyle ou alcoylaminoalcoyle : par libération de la fonction amine du produit correspondant, protégé par exemple par un groupement t.butoxycarbonyle.

— Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, alcoyloxycarbonyl-3 triazol-1,2,4 yle-5 ou triazol-1,2,4 yle-5 substitués en position -1, par sulfoaminoalcoyle : à partir du produit correspondant substitué par un radical t.butoxycarbonylaminoalcoyle, par analogie avec la méthode décrite dans le brevet belge 847 237.

— Lorsque R est un radical alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1, par un radical formylalcoyle ou par un radical de formule générale (XVIa) dans laquelle $R^{\beta}$ représente un atome d'hydrogène : en faisant agir un oxalate d'alcoyle sur une thiosemicarbazide, par analogie avec la méthode décrite par M. Pesson et M. Antoine, C.R. Acad. Sci. 267 (15C), 904 (1968) ou C.R. Acad. Sci. 267 (25C), 1726 (1968).

II. Les thiovinylcéphalosporines de formule générale (XV) dans laquelle R ne contient pas de substituant de formule générale (XVIc), peuvent aussi être préparées par action d'un thiol de formule générale (XXVII) (ou d'un de ses sels alcalins ou alcalino-terreux) dans laquelle R, qui est défini comme ci-dessus, est éventuellement protégé, sur un dérivé de la céphalosporine (ou un mélange des isomères) de formule générale (XIXa), suivie de la réduction du sulfoxyde obtenu (lorsque n = 1) puis de l'élimination des radicaux protecteurs. La protection du radical R du produit de formule générale (XXVII) est la même que définie ci-dessus pour la préparation d'un produit de formule générale (XV) à partir d'un produit de formule générale (XIX) et d'un thiol de formule générale (XXVII).

On opère dans les conditions décrites précédemment pour la réaction des produits de formules générales (XXVII) et (XIX).

Le cas échéant la réduction du S-oxyde, et l'élimination des groupements protecteurs, s'effectuent selon les méthodes décrites précédemment.

III. Les thiovinylcéphalosporines de formule générale (XV) dans laquelle R représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical alcoyle contenant 2 à 4 atomes de carbone substitué par un groupement carbamoyloxy ou acyloxy (dont la partie acyle est éventuellement substituée par un radical amino, alcoylamino ou dialcoylamino), qui sont des dérivés fonctionnels du produit de formule générale (XV) dans laquelle R est un radical —®—alk'—OH choisi parmi dioxo-5,6 hydroxyalcoyl-1 (ou -4) tétrahydro-1,4,5,6 triazine-1,2,4 yle-3, dioxo-5,6 hydroxyalcoyl-2 tétrahydro-1,2,5,6 triazine-1,2,4 yle-3, hydroxyalcoyl-1 triazol-1,3,4 (ou -1,2,4) yle-5, alcoyloxycarbonyl-3 hydroxyalcoyl-1 triazol-1,2,4 yle-5 peuvent être obtenues par carbamatation ou estérification d'un alcool de formule générale :

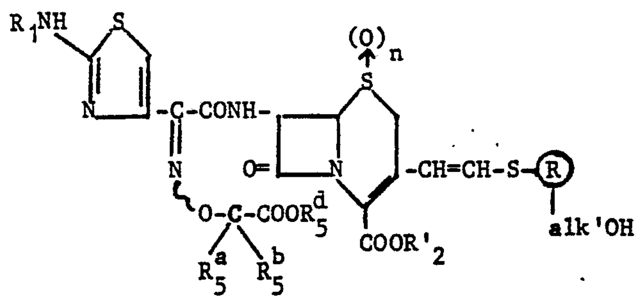

(XVa)

[dans laquelle $R'_2$, $R^a_5$, $R^b_5$, $R^d_5$, $R_1$ et n sont définis comme précédemment et —®—alk'—OH est défini comme ci-dessus] par toute méthode connue pour obtenir un ester ou un carbamate à partir d'un alcool sans toucher au reste de la molécule puis, s'il y a lieu, réduction du sulfoxyde obtenu et élimination des radicaux protecteurs.

L'estérification s'effectue à une température comprise entre − 50 °C et la température de reflux du mélange réactionnel, notamment par condensation de l'anhydride de l'acide (ou d'un autre dérivé réactif, par exemple halogénure) dans un solvant organique interte tel qu'un éther (par exemple tétrahydrofuranne), un solvant chloré (par exemple dichlorométhane), ou un mélange de tels solvants, en présence d'une base azotée comme la pyridine, la diméthylamino-4 pyridine ou une trialcoylamine (triéthylamine) ou d'un agent alcalin de condensation (par exemple bicarbonate de sodium) puis, le cas échéant, réduction du S-oxyde obtenu et élimination des groupements protecteurs selon les méthodes décrites précédemment.

Lorsque le groupement acyloxy substitué par amino ne contient qu'un atome de carbone (groupement carbamoyloxy), on opère notamment par action d'isocyanate de chlorosulfonyle ou de trichloracétyle dans un solvant organique, inerte, par exemple le tétrahydrofuranne ou l'acétonitrile, à une température comprise entre − 80 et 20 °C, puis élimine les groupements protecteurs.

IV. Les thiovinylcéphalosporines de formule générale (XV) dans laquelle R représente un radical

dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical alcoyle contenant 2 à 4 atomes de carbone substitué par un groupement sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido ou dialcoyluréido, ou représente un radical thiadiazol-1,3,4 yle-5 substitué par un radical alcylamino ou acylaminoalcoyle, ou représente un radical oxadiazol-1,3,4 yle-5 substitué par un radical acylaminoalcoyle, ou représente un radical tétrazolyle-5 substitué en position -1 par un radical alcoyle contenant 2 à 4 atomes de carbone substitué par un groupement acylamino, sulfamoylamino, sulfoamino, uréido, alcoyluréido ou dialcoyl-uréido et $R^o_1$ et $R^o_2$ ont les définitions correspondantes, qui sont tous les dérivés fonctionnels de l'amine qui leur correspond, peuvent être obtenues par traitement d'une amine de formule générale :

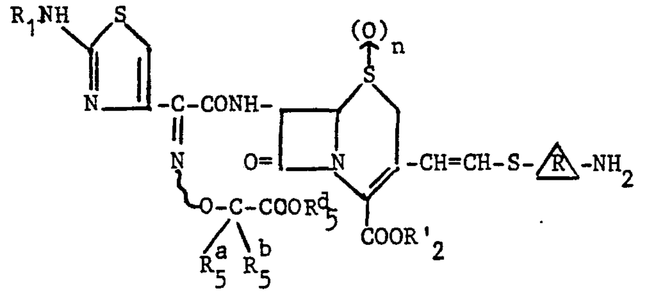

(XVb)

[dans laquelle $R^a_5$, $R^b_5$, $R^d_5$, $R_1$, $R'_2$ et n sont définis comme précédemment pour le produit de formule générale (XVa), et —Ⓡ—$NH_2$ représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical aminoalcoyle dont la partie alcoyle contient 2 à 4 atomes de carbone, ou un radical thiadiazol-1,3,4 yle-5 substitué par un radical amino ou aminoalcoyle, ou un radical oxadiazol-1,3,4 yle-5 substitué par un radical aminoalcoyle, ou un radical tétrazolyle-5 substitué en position -1 par un radical aminoalcoyle dont la partie alcoyle contient 2 à 4 atomes de carbone], par toute méthode connue en soi pour former une fonction amide, sulfamide, carbamate ou urée sans toucher au reste de la molécule, puis le cas échéant réduction du sulfoxyde et élimination des groupements protecteurs.

Il est entendu que les produits qui contiennent un groupement sulfo, sulfonyle ou sulfamoyle sont préparés de préférence à partir d'un produit de formule générale (XVb) dans laquelle n = 0.

Par ailleurs, lorsque l'on veut préparer un produit dont le radical R contient un groupement amino ou hydroxy, il est nécessaire de protéger ces radicaux dans le réactif utilisé.

Lorsque l'on veut préparer un produit de formule générale (XV) dans laquelle le radical R contient un substituant alcoylsulfonylamino, sulfamoylamino, acylamino (substitué ou non), alcoyloxycarbonylamino ou dialcoyluréido, la réaction est effectuée avantageusement par action, respectivement, du dérivé chlorosulfonyle, du chlorure d'acide, de chloroformiate ou du chlorure de dialcoylcarbamoyle correspondant dans les conditions décrites précédemment pour la réaction du chlorure de l'acide de formule générale (X) sur l'amino-7 céphalosporine de formule générale (IX).

Lorsque l'on veut préparer un produit de formule générale (XV) dans laquelle le radical R contient un substituant sulfoamino, alcoylsulfonylamino ou acylamino (substitué ou non), on peut effectuer la réaction au moyen de l'anhydride de l'acide correspondant, dans les conditions décrites précédemment pour faire réagir le produit de formule générale (X) sous forme d'anhydride.

Lorsque l'on veut obtenir un produit de formule générale (XV) pour lequel R contient un radical alcylamino (substitué ou non), il est également possible de faire agir l'acide correspondant, dans les conditions opératoires décrites précédemment pour l'emploi de l'acide de formule générale (X).

Lorsque l'on veut obtenir un produit de formule générale (XV) dans laquelle R contient un radical uréido ou alcoyluréido, on fait agir respectivement un isocyanate alcalin ou un isocyanate d'alcoyle sur le produit correspondant de formule générale (XVb) en milieu hydroorganique ou organique (par exemple dans le tétrahydrofuranne) à une température comprise entre − 20 et 60 °C.

La réduction et l'élimination des radicaux protecteurs s'effectuent dans les conditions décrites précédemment.

V. Les thiovinylcéphalosporines de formule générale (XV) dans laquelle R représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical de formule générale (XVIc) ou par un radical hydroxyiminoalcoyle ou alcoyloxyiminoalcoyle dont la partie iminoalcoyle contient 2 à 5 atomes de carbone ou bien représente un radical tétrazolyl-5 substitué en position -1, par un radical hydroxyiminoalcoyle ou alcoyloxyiminoalcoyle dont la partie iminoalcoyle

contient 2 à 5 atomes de carbone et $R^o_1$ et $R^o_2$ ont les définitions correspondantes, qui sont des dérivés d'addition du produit de formule générale (XV) dans laquelle R est l'un des hétérocycles cités ci-dessus substitué par un radical formylalcoyle (ou sa forme hydrate), peuvent être obtenues à partir d'un aldéhyde de formule générale :

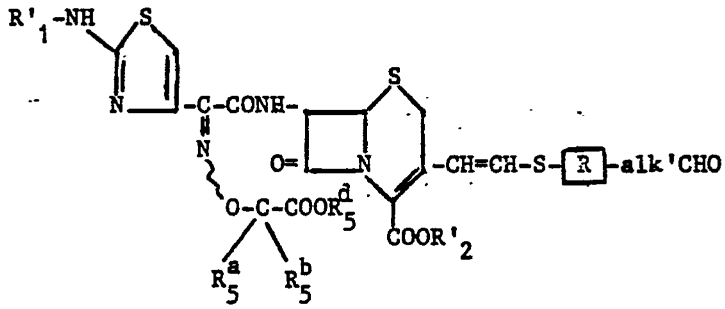

(XVc)

[dans laquelle $R^a_5$, $R^b_5$, $R^d_5$, $R'_1$ et $R'_2$ sont définis comme précédemment et —R—alk'CHO représente un radical dioxo-5,6 formylalcoxy-1 ou -4, tétrahydro-1,4,5,6 triazine-1,2,4 yle-3, formylalcoyl-1 triazol-1,3,4 ou -1,2,4 yle-5, alcoyloxycarbonyl-2 formylalcoyl-1 triazol-1,3,4 yle-5 alcoyloxycarbonyl-3 formylalcoyl-1 triazol-1,2,4 yle-5 ou formylalcoyl-1 tétrazolyle-5], par addition respectivement de cystéamine, d'un alcool, d'hydroxylamine ou d'une alcoyloxyamine selon les méthodes connues pour former des dérivés d'addition de fonctions carbonylées, puis s'il y a lieu élimination des radicaux protecteurs.

La réaction s'effectue généralement dans un solvant organique à une température comprise entre 20 °C et la température de reflux du mélange réactionnel.

Les solvants organiques sont choisis en fonction de la solubilité des produits. Lorsque l'on met en œuvre un produit de formule générale (XVc) dans laquelle $R^d_5$, $R'_1$ et $R'_2$ sont autres que l'hydrogène, on utilise avantageusement des solvants tels que le tétrahydrofuranne, l'acétonitrile, les alcools, les cétones. Lorsque l'on met en œuvre un produit de formule générale (XVc) dans laquelle $R^d_5$, $R'_1$ et $R'_2$ sont des atomes d'hydrogène, on opère avantageusement dans des solvants tels que la pyridine, le diméthylsulfoxyde ou le diméthylformamide.

Lorsque l'on veut préparer un produit de formule générale (XV) pour lequel le radical R contient un substituant de formule générale (XVIc), on opère en milieu acide.

VI. Les thiovinyl-3 céphalosporines de formule générale (XV) dans laquelle $R^o_2$ représente un radical de formule générale (II) dans laquelle $R_6$ et $R_7$ sont définis comme précédemment, peuvent aussi être obtenues par estérification d'un produit de formule générale (XV) dans laquelle $R^o_2$ représente un atome d'hydrogène et dont les fonctions amine et acide du substituant en -7 ont été préalablement protégées, par toute méthode connue en soi pour préparer un ester à partir d'un acide sans toucher au reste de la molécule, puis élimination des groupements protecteurs.

On opère notamment dans les conditions décrites précédemment pour la préparation de produits de formule générale (VII) dans laquelle $R_2$ est un radical de formule générale (II).

Les produits de formules générales (XIX) ou (XIXa), dans lesquelles n = 1 peuvent être obtenus par oxydation des produits correspondants dans lesquels n = 0 selon la méthode décrité dans la demande de brevet DE 2 637 176.

Les isomères des produits de formules générales (I), (XV), (XVII), (XVIII), (XIX) ou (XIXa), peuvent être séparés par cristallisation ou par chromatographie.

Les nouveaux produits selon l'invention et les produits de formule générale (XV) peuvent être éventuellement purifiés par des méthodes physiques telles que la cristallisation, la chromatographie, ou l'ultrafiltration.

Les dérivés de la céphalosporine de formule générale (XV) tels que définis ci-dessus et leurs sels pharmaceutiquement acceptables présentent des propriétés antibactériennes particulièrement intéressantes. Ils manifestent une activité remarquable in vitro et in vivo sur les germes Gram-positifs et Gram-négatifs :

In vitro, ils se sont montrés actifs à une concentration comprise entre 0,5 et 10 $\mu g/cm^3$ sur des souches de staphylocoques sensibles à la pénicilline G (Staphylococcus aureus Smith), à une concentration comprise entre 0,01 et 2 $\mu g/cm^3$ sur Escherichia coli souche NIHJ. De plus, certains se sont montrés actifs à une concentration comprise entre 2 et 125 $\mu g/cm^3$ sur Pseudomonas aeruginosa.

In vivo, ils se sont montrés actifs sur les infections expérimentales de la souris à Staphylococcus aureus Smith (sensible à la pénicilline G) à une dose comprise entre 0,5 et 15 mg/kg par jour par voie sous-cutanée et à Escherichia coli (souche NIHJ) à des doses comprises entre 0,01 et 10 mg/kg par jour par voie sous-cutanée.

Par ailleurs, la $DL_{50}$ des produits de formule générale (XV) est comprise entre 1 g/kg et des doses supérieures à 2,5 g/kg par voie sous-cutanée chez la souris.

L'exemple suivant montre comment l'invention peut être mise en pratique.

Dans cet exemple les produits sont cités selon la nomenclature des Chemical Abstracts. Il est entendu que tous les produits selon la présente invention présentent la stéréochimie donnée par la formule générale partielle :

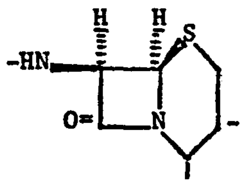

Exemple

A une solution à 80 °C sous azote de 6 g de benzhydryloxycarbonyl-2 [[(t.butoxycarbonyl-2 propyl-2) oxyimino]-2 (tritylamino-2 thiazolyl-4)-2 acétamido]-7 méthyl-3 oxo-8 thia-5 aza-1 bicyclo [4.2.0] octène-2, isomère syn, dans 64 $cm^3$ de diméthylformamide, on ajoute sous agitation 1,9 $cm^3$ de t.butoxy bis-diméthylaminométhane et poursuit la réaction pendant 15 minutes. On verse ensuite le mélange dans un mélange de 200 $cm^3$ d'acétate d'éthyle et 200 $cm^3$ d'eau, décante, lave par 3 fois 100 $cm^3$ d'eau et 100 $cm^3$ d'eau saturée de chlorure de sodium, sèche sur sulfate de sodium, filtre et concentre à sec à 20 °C sous 20 mm de mercure (2,7 kPa). On obtient ainsi 6,5 g de benzhydryloxycarbonyl-2 (diméthylamino-2 vinyl)-3 [[t.butoxycarbonyl-2 propyl-2) oxyimino]-2 (tritylamino-2 thiazolyl-4)-2 acétamido]-7 oxo-8 thia-5 aza-1 bicyclo [4.2.0] octène-2, isomère syn, forme E brut sous la forme d'une meringue brune.

On purifie un échantillon (2,2 g) par chromatographie sur une colonne (diamètre 4 cm, hauteur 20 cm) de gel de silice Merck (0,04-0,06) en éluant par un mélange cyclohexane-acétate d'éthyle 65/35 (vol.) sous une pression de 40 kPa et en recueillant des fractions de 50 $cm^3$. Les fractions 14 à 24 sont concentrées à sec. On obtient 1,2 g de benzhydryloxycarbonyl-2 (diméthylamino-2 vinyl)-3 [[t.butoxycarbonyl-2 propyl-2) oxyimino]-2 (tritylamino-2 thiazolyl-4)-2 acétamido]-7 oxo-8 thia-5 aza-1 bicyclo [4.2.0] octène-2 isomère syn, forme E.

Spectre infrarouge ($CHBr_3$), bandes caractéristiques ($cm^{-1}$) 3 400, 3 280, 1 770, 1 720, 1 680, 1 610, 1 525, 1 490, 1 450, 1 370, 940, 750, 735

Spectre de RMN du proton (350 MHz, $CDCl_3$, δ en ppm, J en Hz) 1,45 (s, 9H, —$C(CH_3)_3$) ; 1,64 et 1,71 (2s, 6H, =N—O—$C(CH_3)_2$—) ; 2,93 (s, 6H, —$N(CH_3)_2$) ; 3,20 et 3,30 (2d, J = 14, 2H, —S—$CH_2$—) ; 5,18 (d, J = 4, 1H, —H en 6) ; 5,71 (dd, J = 4 et 9, 1H, —H en 7) ; 6,61 et 6,82 (2d, J = 14, 2H, —CH=CH—S—) ; 6,88 (s, 1H, H thiazole) ; 6,92 (s, 1H, —COO—CH—$(C_6H_5)_2$) ; 6,92 (s large, 1H, —NH—$C(C_6H_5)_3$); 8,28 (d, J = 9, 1H, —CO—NH—).

Le benzhydryloxycarbonyl-2 [[(t.butoxycarbonyl-2 propyl-2) oxyimino]-2 (tritylamino-2 thiazolyl-4)-2 acétamido]-7 méthyl-3 oxo-8 thia-5 aza-1 bicyclo [4.2.0] octène-2, isomère syn peut être préparé de la manière suivante :

A une solution de 25,58 g d'acide (t.butoxycarbonyl-2 propyl-2 oxyimino)-2 (tritylamino-2 thiazolyl-4)-2 acétique préparé selon le brevet belge 876 541 dans un mélange de 250 $cm^3$ de dichlorométhane et 13,9 $cm^3$ de diméthylacétamide, on ajoute à − 10 °C, en 30 minutes et sous agitation 40 $cm^3$ d'une solution 1,3 M de phosgène dans du chlorobenzène. On agite encore pendant 3 heures à − 10 °C et ajoute goutte à goutte en 1 heure 15 une solution de 16,29 g d'amino-7 benzhydryloxycarbonyl-2 méthyl-3 oxo-8 thia-5 aza-1 bicyclo [4.2.0] octène-2 dans 250 $cm^3$ de dichlorométhane. Après 1 heure 15 à − 10 °C, on lave le mélange par 200 $cm^3$ d'une solution aqueuse à 2 % de bicarbonate de sodium et 200 $cm^3$ d'eau, sèche sur sulfate de sodium et concentre à sec à 30 °C sous 20 mm de mercure (2,7 kPa). Le résidu est chromatographié sur une colonne à 200 g de gel de silice Merck (0,05-0,2) (diamètre de la colonne : 4 cm). On élue par un mélange cyclohexane-acétate d'éthyle 70-30 (en volumes) en recueillant des fractions de 120 $cm^3$. Les fractions 6 à 10 sont concentrées à sec à 30 °C sous 20 mm de mercure (2,7 kPa). On recueille 8,5 g de benzhydryloxycarbonyl-2 [(t.butoxycarbonyl-2 propyl-2 oxyimino)-2 (tritylamino-2 thiazolyl-4)-2 acétamido]-7 méthyl-3 oxo-8 thia-5 aza-1 bicyclo [4.2.0] octène-2, isomère syn, sous la forme d'une meringue jaune.

Spectre infrarouge ($CHBr_3$), bandes caractéristiques ($cm^{-1}$) 3 400, 3 270, 1 790, 1 725, 1 685, 1 525, 1 500, 1 450, 1 380, 1 370, 760, 740.

Les produits selon l'invention peuvent être utilisés de la manière suivante :

Exemple de référence

On agite pendant 1 heure à 20 °C un mélange de 6,45 g de benzhydryloxycarbonyl-2 (diméthylamino-2 vinyl)-3 [[(t.butoxycarbonyl-2 propyl-2) oxyimino]-2 (tritylamino-2 thiazolyl-4)-2 acétamido]-7 oxo-8 thia-5 aza-1 bicyclo [4.2.0] octène-2, isomère syn, forme E en solution dans 100 $cm^3$ d'acétate d'éthyle et 64 $cm^3$ d'acide chlorhydrique 1N. On décante, lave par 100 $cm^3$ d'eau, 100 $cm^3$ d'eau saturée de bicarbonate de sodium et 100 $cm^3$ d'eau saturée de chlorure de sodium, sèche sur sulfate de sodium, filtre et concentre à

sec à 20 °C sous 20 mm de mercure (2,7 kPa). On recueille 4,95 g de benzhydryloxycarbonyl-2 [[[(t.butoxycarbonyl-2 propyl-2) oxyimino]-2 (tritylamino-2 thiazolyl-4)-2 acétamido]]-7 formylméthyl-3 oxo-8 thia-5 aza-1 bicyclo [4.2.0] octène-2, isomère syn brut sous la forme d'une meringue brune.

Spectre infrarouge ($CHBr_3$), bandes caractéristiques ($cm^{-1}$) 3 400, 2 270, 2 720, 1 770, 1 725, 1 685, 1 525, 1 495, 1 450, 1 370, 755, 700

Spectre de RMN du proton (350 MHz, $CDCl_3$, δ en ppm, J en Hz) 1,45 (s, 9H, —$C(CH_3)_3$) ; 1,64 et 1,67 (2s, 6H, (—$CH_3)_2$) ; 3,25 et 3,54 (2d, J = 18, 2H, —$SCH_2$—) ; 3,50 et 3,73 (2d, J = 16, 2H, —$CH_2CHO$) ; 5,10 (d, J = 4, 1H, H en 6) ; 6,06 (dd, J = 4 et 9, 1H, H en 7) ; 6,77 (s, 1H, H du thiazole) ; 6,90 (s, 1H, —COOCH<) ; 8,22 (d, J = 9, 1H, —CONH—) ; 9,58 (s, 1H, —CHO).

A une solution refroidie à 5 °C de 4,45 g de benzhydryloxycarbonyl-2 {[(t.butoxycarbonyl-2 propyl-2) oxyimino]-2 (tritylamino-2 thiazolyl-4)-2 acétamido}-7 formylméthyl-3 oxo-8 thia-5 aza-1 bicyclo [4.2.0] octène-2, isomère syn dans 50 $cm^3$ de pyridine, on ajoute 1,31 g de chlorure de p.toluènesulfonyle. On laisse remonter la température à 20 °C en 30 minutes, agite pendant 1 heure à 20 °C, verse dans 300 $cm^3$ d'eau glacée, décante l'eau et reprend le produit pâteux insoluble dans 300 $cm^3$ d'acétate d'éthyle. On lave par 100 $cm^3$ d'acide chlorhydrique 1N, 100 $cm^3$ d'une solution saturée de bicarbonate de sodium et 100 $cm^3$ d'une solution saturée de chlorure de sodium, sèche sur sulfate de sodium, filtre et concentre à sec à 20 °C sous 20 mm de mercure (2,7 kPa). Le produit ainsi obtenu est utilisé tel quel pour la suite de la synthèse. 1 g de ce produit est purifié par chromatographie sur une colonne de 20 g de gel de silice (0,05-0,2) (diamètre de la colonne : 1,7 cm) et éluant par un mélange de cyclohexane et d'acétate d'éthyle 80-20 (en volumes) et en recueillant des fractions de 50 $cm^3$. Les fractions 4 à 12 contenant le produit pur sont évaporées à sec sous pression réduite (30 mm de mercure ; 4 kPa) à 30 °C. On obtient 0,43 g de benzhydryloxycarbonyl-2 {[(t.butoxycarbonyl-2 propyl-2) oxyimino]-2 (tritylamino-2 thiazolyl-4)-2 acétamido}-7 oxo-8 (tosyloxy-2 vinyl)-3 thia-5 aza-1 bicyclo [4.2.0] octène-2, isomère syn, mélange des formes E et Z (dans des proportions — déterminées par RMN — de 75 % de forme E et 25 % de forme Z) sous la forme d'un solide jaune pâle.

Spectre infrarouge ($CHBr_3$), bandes caractéristiques ($cm^{-1}$) 3 400, 3 260, 1 790, 1 720, 1 680, 1 630, 1 595, 1 580, 1 520, 1 490, 1 450, 1 380, 1 370, 1 190, 1 180, 1 070, 835, 750

Spectre de RMN du proton (350 MHz, $CDCl_3$, δ en ppm, J en Hz) mélange d'isomères E et Z dans les proportions 75/25 ; forme E : 1,45 (s, 9H, —$C(CH_3)_3$) ; 1,62 et 1,67 (2s, 6H, =N—O—$C(CH_3)_2$—) ; 2,48 (s, 3H, —$CH_3$ tosyl) ; 3,42 et 3,50 (2d, J = 18, 2H, —S—$CH_2$—) ; 5,08 (d, J = 4, 1H, —H en 6) ; 6,00 (dd, J = 4 et 9, 1H, —H en 7) ; 6,78 (s, 1H, H thiazole) ; 6,88 (mf, 1H, —NH—$C(C_6H_5)_3$) ; 6,90 et 6,97 (2d, J = 12, 2H, —CH=CH—S) ; 6,92 (s, 1H, —COO—$CH(C_6H_5)_2$ ; 8,20 (d, J = 9, 1H, —CO—NH—) ; forme Z : 6,20 et 6,47 (2d, J = 7, —CH=CH—S— Z) ; 2,45 (s, —$CH_3$ tosyl).

A une solution refroidie à 0 °C de 3 g de benzhydryloxycarbonyl-2 {[(t.butoxycarbonyl-2 propyl-2) oxyimino]-2 (tritylamino-2 thiazolyl-4)-2 acétamido}-7 oxo-8 (tosyloxy-2 vinyl)-3 thia-5 aza-1 bicyclo [4.2.0] octène-2, isomère syn, mélange des formes E et Z (80 % E + 20 % Z) dans 50 $cm^3$ de dichlorométhane, on ajoute goutte à goutte en 25 minutes une solution de 0,464 g d'acide m.chloroperbenzoïque à 90 % dans 10 $cm^3$ de dichlorométhane. On agite pendant 1 heure à 0 °C, dilue par 500 $cm^3$ d'acétate d'éthyle, lave par 2 fois 100 $cm^3$ d'une solution à 2 % de bicarbonate de sodium, 2 fois 100 $cm^3$ d'eau et 100 $cm^3$ d'eau saturée de chlorure de sodium, sèche sur sulfate de sodium, filtre et concentre à sec à 20 °C sous 20 mm de mercure (2,7 kPa). Le résidu est chromatographié sur une colonne de 60 g de silice Merck (0,06-0,2) (diamètre de la colonne : 2 cm, hauteur : 20 cm), on élue par 1 litre d'un mélange cyclohexanne-acétate d'éthyle (70-30 en volumes) en recueillant des fractions de 60 $cm^3$. Les fractions 5 à 14 sont concetrées à sec à 20 °C sous 20 mm de mercure (2,7 kPa), on recueille 1,9 g de benzhydryloxycarbonyl-2 [[[(t.butoxycarbonyl-2 propyl-2) oxyimino]-2 (tritylamino-2 thiazolyl-4)-2 acétamido]]-7 oxo-8 oxyde-5 (tosyloxy-2 vinyl)-3 thia-5 aza-1 bicyclo [4.2.0] octène-2, isomère syn, forme E sous la forme d'une meringue jaune.

Spectre infrarouge ($CHBr_3$), bandes caractéristiques ($cm^{-1}$) 3 380, 1 800, 1 720, 1 680, 1 590, 1 580, 1 510, 1 490, 1 445, 1 375, 1 190, 1 175, 1 070, 730

Spectre de RMN du proton (350 MHz, DMSO $d_6$, δ en ppm, J en Hz) 1,37 (s, 9H, —$C(CH_3)_3$) ; 1,45 et 1,46 (2s, 6H, —O—$C(CH_3)_2$—) ; 2,44 (s, 3H, —$CH_3$ tosyle) ; 3,60 et 4,41 (2d, J = 18, 2H, —$SCH_2$—) ; 5,06 (d, J = 4, 1H, H en 6) ; 5,96 (dd, J = 4 et 9, 1H, H en 7) ; 6,75 (d, J = 13, 1H, —CH=CHS—) ; 6,73 (s, 1H, H du thiazole) ; 6,93 (s, 1H, —COOCH—) ; 7,48 et 7,84 (ab, 2H, J = 9) ; 8,16 (d, J = 9, 1H, —CONH—) ; 8,73 (s, 1H, —NH—$C(C_6H_5)_3$).

On chauffe à 60 °C pendant 4 heures sous agitation et sous azote un mélange de 6,79 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo [4.2.0] octène-2, isomère syn, forme E, 60 $cm^3$ de diméthylformamide, 1,68 g de (diméthoxy-2,2 éthyl)-4 dioxo-5,6 thioxo-3 perhydrotriazine-1,2,4 et 1,25 $cm^3$ de N,N-diisopropyléthylamine. On dilue le mélange par 250 $cm^3$ d'acétate d'éthyle, lave par 3 fois 125 $cm^3$ d'eau et 2 fois 125 $cm^3$ d'une solution saturée de chlorure de sodium, sèche sur sulfate de sodium, filtre et concentre à sec à 20 °C sous 20 mm de mercure (2,7 kPa). Le résidu obtenu après ce traitement est chromatographié sur une colonne de 125 g de gel de silice Merck (0,06-0,2) (diamètre de la colonne : 3 cm, hauteur : 43 cm). On élue par 1,5 litre d'un mélange cyclohexaneacétate d'éthyle 50-50 (en volumes) et 1 litre d'acétate d'éthyle en recueillant des fractions de 100 $cm^3$. Les fractions 16 à 21 sont concentrées à sec à 25 °C sous 20 mm de mercure (2,7 kPa), on obtient 5,5 g de benzhydryloxycarbonyl-2

{[(t.butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, isomère syn, forme E sous la forme d'une meringue brune.

Spectre infrarouge ($CHBr_3$), bandes caractéristiques ($cm^{-1}$) 1 800, 1 720, 1 690, 1 585, 1 510, 1 495, 1 445, 1 370, 1 080, 1 060, 1 040, 940, 750, 700

Spectre de RMN du proton (350 MHz, DMSO $d_6$, δ en ppm, J en Hz) 1,35 (s, 9H, $—C(CH_3)_3$) ; 1,44 et 1,45 (2s, 6H, $=N—O—C(CH_3)_2—$) ; 3,32 (s, 6H, $(—OCH_3)_2$) ; 3,65 et 4,36 (2d, J = 18, 2H, $—S—CH_2—$) ; 3,95 (d, J = 5, 2H, $>N—CH_2—$) ; 4,56 (t, J = 5, 1H, 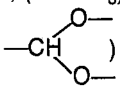 ) ; 5,09 (d, J = 5, 1H, —H en 6) ; 5,95 (dd, J = 5 et 9, 1H, —H en 7) ; 6,78 (s, 1H, —H thiazole) ; 7 (s, 1H, $—COO—CH(C_6H_5)_2$) ; 7,02 et 7,1 (2d, J = 16, 2H, —CH=CH—S—) ; 8,23 (d, J = 9, 1H, —CONH—) ; 8,73 (s, 1H, $—NH—C(C_6H_5)_3$) ; 12,65 (s, 1H, =N—NH—CO— ou =N—N=C—OH).

On traite à − 5 °C pendant 1 h 30 sous agitation, une solution de 5,37 g de benzhydryloxycarbonyl-2 [[(t.butoxycarbonyl-2 propyl-2) oxyimino]-2 (tritylamino-2 thiazolyl-4)-2 acétamido]-7 [[(diméthoxy-2,2 éthyl)-4 dioxo-5,6 tétrahydro-1,4,5,6 triazine-1,2,4 yl-3] thio-2 vinyl]-3 oxo-8 oxyde-5 thia-5 aza-1 bicyclo [4.2.0] octène-2, isomère syn, forme E dans 45 $cm^3$ de dichlorométhane et 1,79 $cm^3$ de N,N-diméthylacétamide par 0,79 $cm^3$ de trichlorure de phosphore. Après lavage du mélange réactionnel avec une solution à 2 % de bicarbonate de sodium et évaporation à sec, le résidu est dissous dans le minimum de dichlorométhane et chromatographié sur une colonne de 80 g de gel de silice Merck (0,06-0,2) (diamètre de la colonne : 2 cm, hauteur : 20 cm), on élue par 250 $cm^3$ d'un mélange cyclohexane-acétate d'éthyle 40-60 (en volumes) et 1,5 litre d'un mélange 30-70 en recueillant des fractions de 100 $cm^3$. Les fractions 5 à 14 sont concentrées à sec à 25 °C sous 20 mm de mercure, on obtient 4,02 g de benzhydryloxycarbonyl-2 [[(t.butoxycarbonyl-2 propyl-2) oxyimino]-2 (tritylamino-2 thiazolyl-4)-2 acétamido]-7 [[(diméthoxy-2,2 éthyl)-4 dioxo-5,6 tétrahydro-1,4,5,6 triazine-1,2,4 yl-3] thio-2 vinyl]-3 oxo-8 thia-5 aza-1 bicyclo [4.2.0] octène-2, isomère syn, forme E sous la forme d'une meringue brun clair.

Spectre infrarouge (KBr), bandes caractéristiques ($cm^{-1}$) 1 790, 1 720, 1 690, 1 585, 1 520, 1 495, 1 450, 1 370, 1 080, 940, 750, 700

Spectre de RMN du proton (350 MHz, DMSO $d_6$, δ en ppm, J en Hz) 1,37 (s, 9H, $—C(CH_3)_3$) ; 1,42 (s, 6H, $=N—O—C(CH_3)_2—$) ; 3,31 (s, 6H, $(—OCH_3)_2$) ; 3,64 et 3,89 (2d, J = 18, 2H, $—S—CH_2—$) ; 3,95 (d, J = 5, 2H, $>N—CH_2—$) ; 4,56 (t, J = 5, 1H, 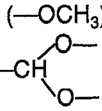 ) ; 5,26 (d, J = 4, 1H, —H en 6) ; 5,77 (dd, J = 4 et 9, 1H, —H en 7) ; 6,71 (s, 1H, —H thiazole) ; 6,90 et 7,03 (2d, J = 16, 2H, —CH=CH—S—) ; 6,97 (s, 1H, $—COO—CH(C_6H_5)_2$) ; 8,80 (s, 1H, $—NH—C(C_6H_5)_3$) ; 9,39 (d, J = 9, 1H, —CO—NH—) ; 12,66 (s, 1H, =N—NH—CO— ou =N—N=C—OH).

On agite à 20 °C pendant 20 minutes une solution de 3,89 g de benzhydryloxycarbonyl-2 [[(t.butoxycarbonyl-2 propyl-2) oxyimino]-2 (tritylamino-2 thiazolyl-4)-2 acétamido]-7 [[(diméthoxy-2,2 éthyl)-4 dioxo-5,6 tétrahydro-1,4,5,6 triazine-1,2,4 yl-3] thio-2 vinyl]-3 oxo-8 thia-5 aza-1 bicyclo [4.2.0] octène-2, isomère syn, forme E dans 39 $cm^3$ d'acide trifluoracétique. On concentre à sec à 20 °C sous 0,05 mm de mercure (0,007 kPa), reprend le résidu dans 100 $cm^3$ d'éther diéthylique, agite pendant 10 minutes et filtre. Le solide obtenu est traité à 50 °C pendant 45 minutes par 80 $cm^3$ d'acide formique, on ajoute 16 $cm^3$ d'eau, maintient 30 minutes à 50 °C et concentre à sec à 20 °C sous 0,05 mm de mercure (0,007 kPa). On reprend le résidu par 3 fois 150 $cm^3$ d'acétone en évaporant à chaque fois à 20 °C sous 30 mm de mercure (4 kPa) et chauffe le résidu à reflux sous agitation dans 100 $cm^3$ d'acétone. On filtre et recueille 2,15 g d'[[(amino-2 thiazolyl-4)-2 [(carboxy-2 propyl-2) oxyimino]-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2, isomère syn, forme E sous la forme d'une poudre jaune.

Spectre infrarouge (KBr), bandes caractéristiques ($cm^{-1}$) 3 400, 3 300, 3 200, 2 200, 1 780, 1 720, 1 685, 1 585, 1 540, 1 000

Spectre de RMN du proton (350 MHz, $CF_3COOD$, δ en ppm, J en Hz) 1,85 et 1,86 (2s, 6H, $—CH_3$) ; 3,90 (s large, 2H, $—SCH_2—$) ; 5,20 (s, 2H, $—CH_2CHO$) ; 5,40 (d, J = 4, 1H, H en 6) ; 6,12 (d, J = 4, 1H, H en 7) ; 7,23 et 7,76 (2d, J = 16, 2H, —CH=CH—) ; 7,50 (s, 1H, H du thiazole) ; 9,73 (s, 1H, —CHO).

**Revendications** (pour les Etats contractants : BE, CH, DE, FR, GB, IT, LI, LU, NL, SE)

1. Un dérivé de vinyl-3 céphalosporine, caractérisé en ce qu'il répond à la formule générale :

(Voir dessin, page 20)

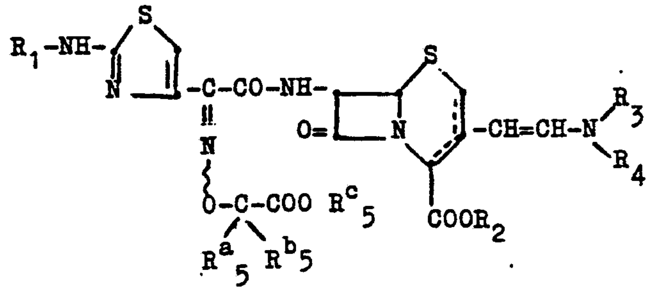

qui se présente sous forme bicyclooctène-2 ou -3 et dans laquelle

— le substituant en position -3 du bicyclooctène présente la stéréoisomérie E ou Z ;

— le groupe imine du substituant en position 7 se présente sous forme syn ou anti ;

— les radicaux $R^a_5$ et $R^b_5$, qui sont identiques ou différents, représentent des atomes d'hydrogène ou des radicaux alcoyle, ou forment ensemble un radical alcoylène contenant 2 ou 3 atomes de carbone ;

— le symbole $R^c_5$ représente un radical protecteur d'acide ;

— le symbole $R_1$ représente un radical protecteur d'amine ;

— le symbole $R_2$ représente un radical facilement éliminable par voie enzymatique de formule générale :

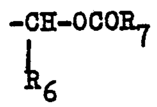

⟦dans laquelle $R_6$ représente un atome d'hydrogène ou un radical alcoyle et $R_7$ représente un radical alcoyle ou le radical cyclohexyle⟧, ou représente un radical méthoxyméthyle, t.butyle, benzhydryle, p.nitrobenzyle ou p.méthoxybenzyle ; et

— les symboles $R_3$ et $R_4$, qui sont identiques ou différents, représentent des radicaux alcoyle (éventuellement substitués par un radical hydroxy, alcoyloxy, amino, alcoylamino ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, les portions ou radicaux alcoyle cités ci-dessus étant (sauf mention spéciale) droits ou ramifiés et contenant 1 à 4 atomes de carbone, ainsi que les mélanges de leurs isomères.

2. Un dérivé de vinyl-3 céphalosporine selon la revendication 1, dans la formule duquel le radical protecteur $R^c_5$ est choisi parmi méthoxyméthyle, t.butyle, benzhydryle, benzyle, nitrobenzyle et p.méthoxybenzyle.

3. Un dérivé de vinyl-3 céphalosporine, selon la revendication 1, dans la formule duquel le radical protecteur $R_1$ est choisi parmi t.butoxycarbonyle, trichloro-2,2,2 éthoxycarbonyle, chloracétyle, trichloroacétyle, trityle, benzyle, dibenzyle, benzyloxycarbonyle, p.nitrobenzyloxycarbonyle, p.méthoxybenzyloxycarbonyle, formyle et trifluoroacétyle.

4. Procédé de préparation d'un produit selon la revendication 1 [pour lequel $R_3$ et $R_4$ sont définis comme dans la revendication 1, à l'exception de représenter alcoyle substitué par hydroxy, amino ou alcoylamino, et les autres symboles sont définis selon la revendication 1, caractérisé en ce que l'on fait agir un produit de formule générale :

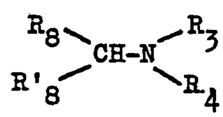

[dans laquelle $R_3$ et $R_4$ sont définis comme ci-dessus et $R_8$ et $R'_8$ qui sont identiques ou différents, soit représentent des groupements de formule générale :

$$-X_2R_9$$

dans laquelle $X_2$ est l'oxygène et $R_9$ est un radical alcoyle contenant 1 à 5 atomes de carbone ou phényle, soit représentent chacun un radical amino de formule générale :

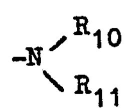

dans laquelle $R_{10}$ et $R_{11}$ sont définis comme $R_3$ et $R_4$, soit encore représentent l'un un radical $-X_2R_9$ dans lequel $X_2$ est l'oxygène ou le soufre et l'autre un radical $-NR_{10}R_{11}$] sur un dérivé de la céphalosporine de formule générale :

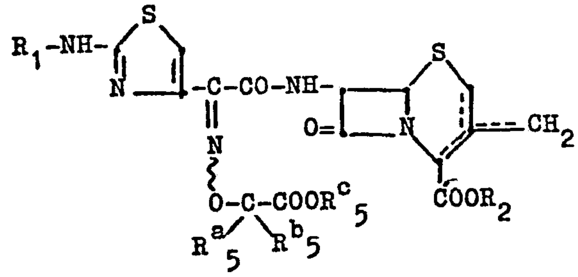

qui se présente sous forme bicyclooctène-2 ou -3 ou méthylène-3 bicyclooctène et dans laquelle les différents symboles sont définis selon la revendication 1 puis on sépare éventuellement le produit obtenu en ses isomères.

5. Procédé selon la revendication 4, caractérisé en ce que l'on fait agir un produit de formule générale :

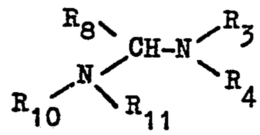

dans laquelle le radical $-NR_{10}R_{11}$ est le reste d'une amine plus volatile que $HNR_3R_4$.

6. Procédé de préparation d'un produit selon la revendication 1 [pour lequel $R_3$ et $R_4$, qui sont identiques ou différents, représentent des radicaux alcoyle substitués par hydroxy, amino ou alcoylamino et les autres symboles sont définis comme dans la revendication 1], caractérisé en ce que l'on effectue la transénamination d'un produit selon la revendication 1 [pour lequel $R_3$ et $R_4$ représentent des radicaux alcoyle et les autres symboles sont définis selon la revendication 1], par action d'une amine de formule générale :

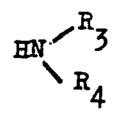

dans laquelle $R_3$ et $R_4$, qui sont identiques ou différents, représentent des radicaux alcoyle substitués par hydroxy, amino ou alcoylamino, puis on sépare éventuellement le produit obtenu en ses isomères.

**Revendication** (pour l'Etat contractant AT)

Procédé de préparation d'un dérivé de vinyl-3 céphalosporine de formule générale :

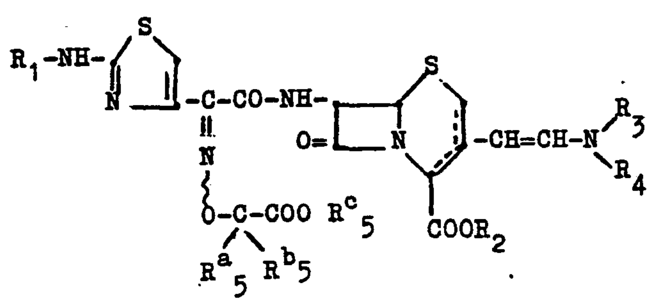

qui se présente sous forme bicyclooctène-2 ou -3 et dans laquelle

— le substituant en position -3 du bicyclooctène présente la stéréoisomérie E ou Z ;

— le groupe imine du substituant en position 7 se présente sous forme syn ou anti ;

— les radicaux $R^a_5$ et $R^b_5$, qui sont identiques ou différents, représentent des atomes d'hydrogène ou des radicaux alcoyle, ou forment ensemble un radical alcoylène contenant 2 ou 3 atomes de carbone ;

— le symbole $R^c_5$ représente un radical protecteur d'acide ;

— le symbole $R_1$ représente un radical protecteur d'amine ;

— le symbole $R_2$ représente un radical facilement éliminable par voie enzymatique de formule générale :

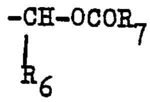

[[dans laquelle $R_6$ représente un atome d'hydrogène ou un radical alcoyle et $R_7$ représente un radical alcoyle ou le radical cyclohexyle]], ou représente un radical méthoxyméthyle, t.butyle, benzhydryle, p.nitrobenzyle ou p.méthoxybenzyle ; et

— les symboles $R_3$ et $R_4$, qui sont identiques ou différents, représentent des radicaux alcoyle (éventuellement substitués par un radical hydroxy, alcoyloxy, amino, alcoylamino ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, les portions ou radicaux alcoyle cités ci-dessus étant (sauf mention spéciale) droits ou ramifiés et contenant 1 à 4 atomes de carbone, ainsi que les mélanges de leurs isomères, caractérisé en ce que pour la préparation d'un produit dans la formule duquel $R_3$ et $R_4$ sont définis comme ci-dessus, à l'exception de représenter alcoyle substitué par hydroxy, amino ou alcoylamino, on fait agir un produit de formule générale :

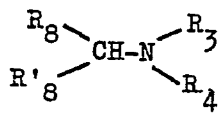

[dans laquelle $R_3$ et $R_4$ sont définis comme ci-dessus et $R_8$ et $R'_8$, qui sont identiques ou différents, soit représentent des groupements de formule générale :

$$—X_2R_9$$

dans laquelle $X_2$ est l'oxygène et $R_9$ est un radical alcoyle contenant 1 à 5 atomes de carbone ou phényle, soit représentent chacun un radical amino de formule générale :

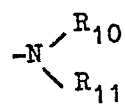

dans laquelle $R_{10}$ et $R_{11}$ sont définis comme $R_3$ et $R_4$, soit encore représentent l'un un radical $—X_2R_9$ dans lequel $X_2$ est l'oxygène ou le soufre et l'autre un radical $—NR_{10}R_{11}$] sur un dérivé de la céphalosporine de formule générale :

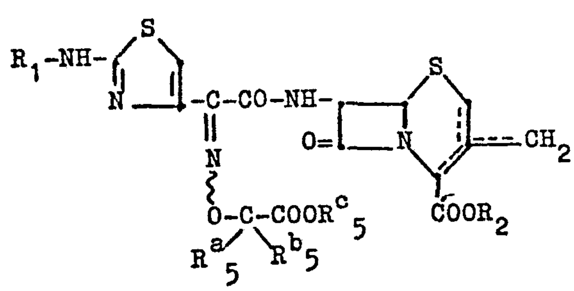

qui se présente sous forme bicyclooctène-2 ou -3 ou méthylène-3 bicyclooctane et dans laquelle les différents symboles sont définis comme ci-dessus, puis on sépare éventuellement le produit obtenu en ses isomères ou, pour la préparation d'un produit dans la formule duquel $R_3$ et $R_4$, qui sont identiques ou différents, représentent des radicaux alcoyle substitués par hydroxy, amino ou alcoylamino, on effectue la transénamination d'un produit précédemment obtenu [pour lequel $R_3$ et $R_4$ représentent des radicaux alcoyle et les autres symboles sont définis comme ci-dessus], par action d'une amine de formule générale :

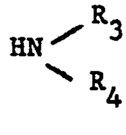

dans laquelle $R_3$ et $R_4$, qui sont identiques ou différents, représentent des radicaux alcoyle substitués par hydroxy, amino ou alcoylamino, puis on sépare éventuellement le produit obtenu en ses isomères.

**Claims** (for the Contracting States : BE, CH, DE, FR, GB, IT, LI, LU, NL, SE)

1. A 3-vinylcephalosporin derivative, characterised in that it corresponds to the general formula :

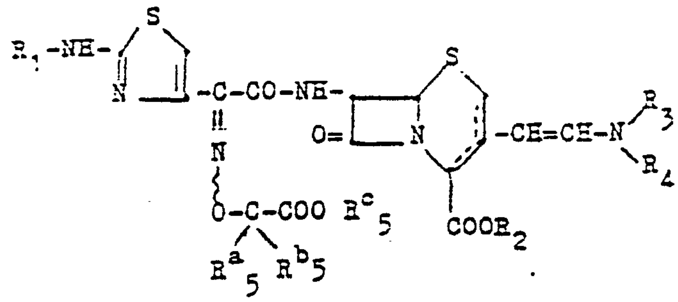

which is in the form of a bicyclooct-2-ene or bicyclooct-3-ene and in which

— the substituent in the 3-position of the bicyclooctene exhibits E or Z stereoisomerism ;

— the imine group of the substituent in the 7-position is in the syn or anti form ;

— the radicals $R^a_5$ and $R^b_5$, which are identical or different, represent hydrogen atoms or alkyl radicals or together form an alkylene radical containing 2 or 3 carbon atoms ;

— the symbol $R^c_5$ represents an acid-protecting radical ;

— the symbol $R_1$ represents an amine-protecting radical ;

— the symbol $R_2$ represents a radical which can easily be removed by an enzymatic method, of the general formula :

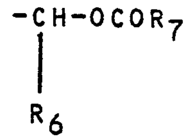

⟦in which $R_6$ represents a hydrogen atom or an alkyl radical and $R_7$ represents an alkyl radical or the cyclohexyl radical⟧, or represents a methoxymethyl, t-butyl, benzhydryl, p-nitrobenzyl or p-methoxybenzyl radical ; and

— the symbols $R_3$ and $R_4$, which are identical or different, represent alkyl radicals (optionally substituted by a hydroxyl, alkoxy, amino, alkylamino or dialkylamino radical) or phenyl radicals or together form, with the nitrogen atom to which they are attached, a saturated 5-membered or 6-membered heterocycle optionally containing another heteroatom chosen from amongst nitrogen, oxygen or sulphur, and optionally substituted by an alkyl radical, the above-mentioned alkyl portions or radicals being linear or branched (unless otherwise mentioned) and containing 1 to 4 carbon atoms, and also mixtures of its isomers.

2. A 3-vinylcephalosporin derivative according to Claim 1, in the formula of which the protecting radical $R^c_5$ is chosen from amongst methoxymethyl, t-butyl, benzhydryl, benzyl, nitrobenzyl and p-methoxybenzyl.

3. A 3-vinylcephalosporin derivative according to Claim 1, in the formula of which the protecting radical $R_1$ is chosen from amongst t-butoxycarbonyl, 2,2,2-trichloroethoxycarbonyl, chloroacetyl, trichloroacetyl, trityl, benzyl, dibenzyl, benzyloxycarbonyl, p-nitrobenzyloxycarbonyl, p-methoxybenzyloxycarbonyl, formyl and trifluoroacetyl.

4. Process for the preparation of a product according to Claim 1 [in which $R_3$ and $R_4$ are defined as in Claim 1, except that they cannot represent alkyl substituted by hydroxyl, amino or alkylamino, and the other symbols are defined according to Claim 1], characterised in that a product of the general formula :

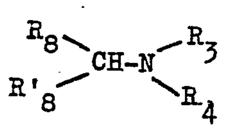

[in which $R_3$ and $R_4$ are defined as above and $R_8$ and $R'_8$, which are identical or different, represent groups of the general formula :

$$—X_2R_9$$

in which $X_2$ is oxygen and $R_9$ is an alkyl radical contraining 1 to 5 carbon atoms or a phenyl radical, or each represent an amino radical of the general formula :

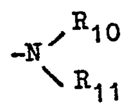

in which $R_{10}$ and $R_{11}$ are defined in the same way as $R_3$ and $R_4$, or alternatively represent in one case a radical $—X_2R_9$, in which $X_2$ is oxygen or sulphur, and in the other case a radical $—NR_{10}R_{11}$] is reacted with a cephalosporin derivative of the general formula :

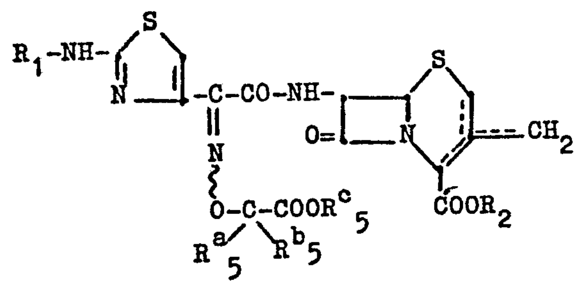

which is in the form of a bicyclooct-2-ene or bicyclooct-3-ene or a 3-methylenebicyclooctane and in which the various symbols are defined according to Claim 1, and the product obtained is then separated into its isomers, if appropriate.

5. Process according to Claim 4, characterised in that a product of the general formula :

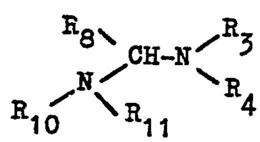

in which the radical $—NR_{10}R_{11}$ is the radical of an amine which is more volatile than $HNR_3R_4$, is reacted.

6. Process for the preparation of a product according to Claim 1 [in which $R_3$ and $R_4$, which are identical or different, represent alkyl radicals substituted by hydroxyl, amino or alkylamino and the other symbols are defined as in Claim 1], characterised in that a product according to Claim 1 [in which $R_3$ and $R_4$ represent alkyl radicals and the other symbols are defined according to Claim 1] is subjected to transenamination by reaction with an amine of the general formula :

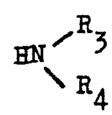

in which $R_3$ and $R_4$, which are identical or different, represent alkyl radicals substituted by hydroxyl, amino or alkylamino, and the product obtained is then separated into its isomers, if appropriate.

**Claim** (for the Contracting State AT)

Process for the preparation of a 3-vinylcephalosporin derivative of the general formula :

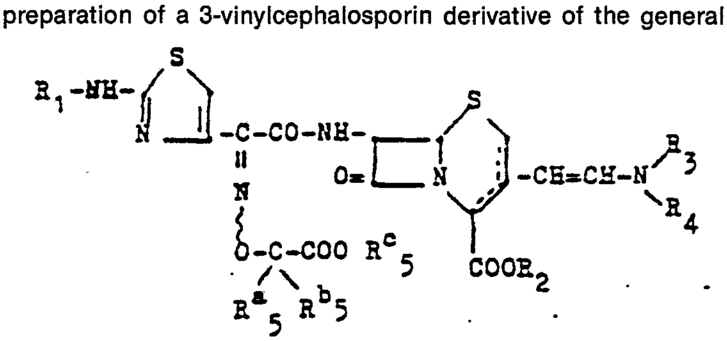

which is in the form of a bicyclooct-2-ene or bicyclooct-3-ene and in which

— the substituent in the 3-position of the bicyclooctene exhibits E or Z stereoisomerism ;

— the imine group of the substituent in the 7-position is in the syn or anti form ;

— the radicals $R^a_5$ and $R^b_5$, which are identical or different, represent hydrogen atoms or alkyl radicals or together form an alkylene radical containing 2 or 3 carbon atoms ;

— the symbol $R^c_5$ represents an acid-protecting radical ;

— the symbol $R_1$ represents an amine-protecting radical ;

— the symbol $R_2$ represents a radical which can easily be removed by an enzymatic method, of the general formula :

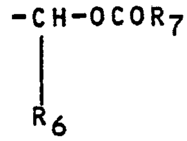

[in which $R_6$ represents a hydrogen atom or an alkyl radical and $R_7$ represents an alkyl radical or the cyclohexyl radical], or represents a methoxymethyl, t-butyl, benzhydryl, p-nitrobenzyl or p-methoxybenzyl radical ; and

— the symbols $R_3$ and $R_4$, which are identical or different, represent alkyl radicals (optionally substituted by a hydroxyl, alkoxy, amino, alkylamino or dialkylamino radical) or phenyl radicals or together form, with the nitrogen atom to which they are attached, a saturated 5-membered or 6-membered heterocycle optionally containing another heteroatom chosen from amongst nitrogen, oxygen or sulphur, and optionally substituted by an alkyl radical, the abovementioned alkyl portions or radicals being linear or branched (unless otherwise mentioned) and containing 1 to 4 carbon atoms, and also mixtures of its isomers, characterised in that, to prepare a product in the formula of which $R_3$ and $R_4$ are defined as above, except that they cannot represent alkyl substituted by hydroxyl, amino or alkylamino, a product of the general formula :

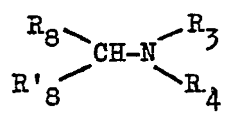

[in which $R_3$ and $R_4$ are defined as above and $R_8$ and $R'_8$, which are identical or different, represent groups of the general formula :

$$—X_2R_9$$

in which $X_2$ is oxygen and $R_9$ is an alkyl radical containing 1 to 5 carbon atoms or a phenyl radical, or each represent an amino radical of the general formula :

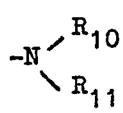

in which $R_{10}$ and $R_{11}$ are defined in the same way as $R_3$ and $R_4$, or alternatively represent in one case a radical $—X_2R_9$, in which $X_2$ is oxygen or sulphur, and in the other case a radical $—NR_{10}R_{11}$] is reacted with a cephalosporin derivative of the general formula :

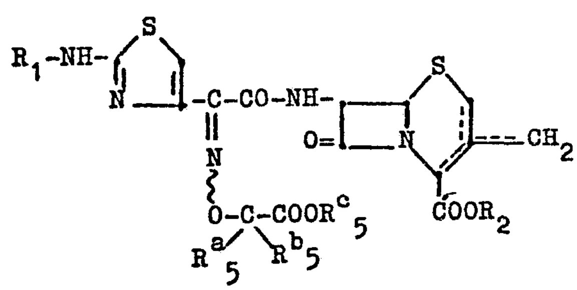

which is in the form of a bicyclooct-2-ene or bicyclooct-3-ene or a 3-methylenebicyclooctane and in which the various symbols are defined as above, and the product obtained is then separated into its isomers, if appropriate, or, to prepare a product in the formula of which $R_3$ and $R_4$, which are identical or different, represent alkyl radicals substituted by hydroxyl, amino or alkylamino, a product previously obtained [in which $R_3$ and $R_4$ represent alkyl radicals and the other symbols are defined as above] is subjected to transenamination by reaction with an amine of the general formula :

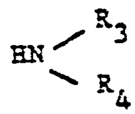

in which $R_3$ and $R_4$, which are identical or different, represent alkyl radicals substituted by hydroxyl, amino or alkylamino, and the product obtained is then separated into its isomers, if appropriate.

**Ansprüche** (für die Vertragsstaaten : BE, CH, DE, FR, GB, IT, LI, LU, NL, SE)

1. Derivat von 3-Vinylcephalosporin, dadurch gekennzeichnet, daß es der allgemeinen Formel :

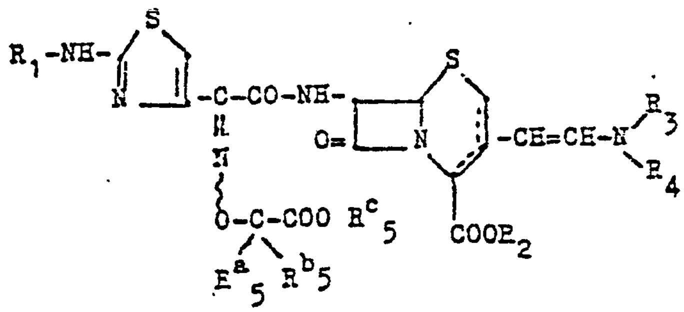

entspricht, welches in Form von Bicycloocten-2 oder -3 vorliegt, worin

— der Substituent in 3-Stellung des Bicyclooctens die E- oder Z-Stereoisomerie aufweist ;

— die Iminogruppe des Substituenten in 7-Stellung in syn- oder anti-Form vorliegt ;

— die Reste $R^a_5$ und $R^b_5$, welche identisch oder voneinander verschieden sind, Wasserstoffatome oder Alkylreste bedeuten oder zusammen einen Alkylenrest mit 2 oder 3 Kohlenstoffatomen bilden ;

— das Symbol $R^c_5$ einen Säureschutzrest bedeutet ;

— das Symbol $R_1$ einen Aminschutzrest bedeutet ;

— das Symbol $R_2$ einen leicht auf enzymatischem Wege entfernbaren Rest der allgemeinen Formel :

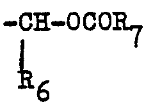

darstellt [[worin $R_6$ ein Wasserstoffatom oder einen Alkylrest und $R_7$ einen Alkylrest oder den Cyclohexylrest bedeuten]] oder einen Methoxymethyl-, tert.-Butyl-, Benzhydryl-, p-Nitrobenzyl- oder p-Methoxybenzylrest bedeutet ; und

— die Symbole $R_3$ und $R_4$, welche identisch oder verschieden sind, Alkylreste (gegebenenfalls substituiert durch einen Hydroxy-, Alkyloxy-, Amino-, Alkylamino- oder Dialkylaminorest) oder Phenyl bedeuten oder zusammen mit dem Stickstoffatom, an das sie gebunden sind, einen gesättigten Heterocyclus mit 5 oder 6 Kettengliedern, enthaltend gegebenenfalls ein weiteres Heteroatom, ausgewählt unter Stickstoff, Sauerstoff oder Schwefel, und gegebenenfalls substituiert durch einen Alkylrest, bilden, wobei die oben erwähnten Alkylteile oder -reste (wenn nichts anderes angegeben ist) geradkettig oder verzweigt sind und 1 bis 4 Kohlenstoffatome enthalten, sowie die Gemische ihrer Isomeren.

2. 3-Vinyl-cephalosporin-Derivat gemäß Anspruch 1, in dessen Formel der Schutzrest $R^c_5$ ausgewählt ist unter Methoxymethyl, tert.-Butyl, Benzhydryl, Benzyl, Nitrobenzyl und p-Methoxybenzyl.

3. 3-Vinyl-cephalosporin-Derivat gemäß Anspruch 1, in dessen Formel der Schutzrest $R_1$ ausgewählt ist unter tert.-Butoxycarbonyl, 2,2,2-Trichlor-ethoxycarbonyl, Chloracetyl, Trichloracetyl, Trityl, Benzyl, Dibenzyl, Benzyloxycarbonyl, p-Nitrobenzyloxycarbonyl, p-Methoxybenzyloxycarbonyl, Formyl und Trifluoracetyl.

4. Verfahren zur Herstellung eines Produktes gemäß Anspruch 1 [worin $R_3$ und $R_4$ wie in Anspruch 1 definiert sind, mit Ausnahme der Bedeutung von Alkyl, substituiert durch Hydroxy, Amino oder Alkylamino, und die anderen Symbole gemäß Anspruch 1 definiert sind], dadurch gekennzeichnet, daß man ein Produkt der allgemeinen Formel :

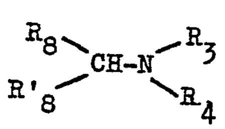

[worin $R_3$ und $R_4$ wie oben definiert sind und $R_8$ und $R'_8$, welche identisch oder verschieden sind, entweder Gruppen der allgemeinen Formel :

$$—X_2R_9$$

bedeuten, worin $X_2$ Sauerstoff ist und $R_9$ einen Alkylrest mit 1 bis 5 Kohlenstoffatomen oder Phenyl darstellt, oder jeweils einen Aminorest der allgemeinen Formel :

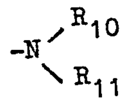

darstellt, worin $R_{10}$ und $R_{11}$ wie für $R_3$ und $R_4$ definiert sind oder auch der eine einen Rest $-X_2R_9$ darstellt, worin $X_2$ Sauerstoff oder Schwefel ist und der andere ein Rest $—NR_{10}R_{11}$ ist] mit einem Derivat des Cephalosporins der allgemeinen Formel :

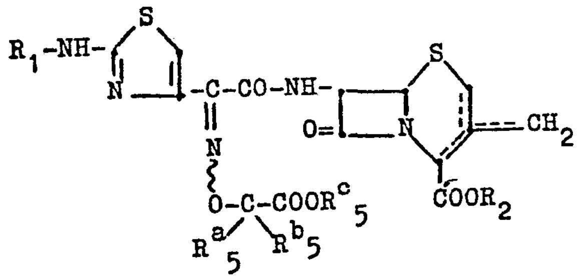

umsetzt, das in Form von Bicycloocten-2 oder 3- oder 3-Methylen-bicyclooctan vorliegt und worin die verschiedenen Symbole wie in Anspruch 1 definiert sind, und daß man dann gegebenenfalls das erhaltene Produkt in seine Isomeren auftrennt.

5. Verfahren gemäß Anspruch 4, dadurch gekennzeichnet, daß man ein Produkt der allgemeinen Formel :

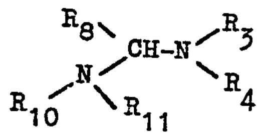

worin der Rest $-NR_{10}R_{11}$ der Rest eines Amins ist, das flüchtiger als $HNR_3R_4$ ist.

6. Verfahren zur Herstellung eines Produktes gemäß Anspruch 1 [worin $R_3$ und $R_4$, welche identisch oder verschieden sind, Alkylreste, substituiert durch Hydroxy, Amino oder Alkylamino, bedeuten und die anderen Symbole wie in Anspruch 1 definiert sind], dadurch gekennzeichnet, daß man die Trans-Enaminierung eines Produktes gemäß Anspruch 1 [wofür $R_3$ und $R_4$ Alkylreste darstellen und die anderen Symbole wie in Anspruch 1 definiert sind] durch Einwirkung eines Amins der allgemeinen Formel :

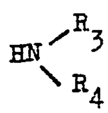

bewirkt, worin $R_3$ und $R_4$, welche identisch oder verschieden sind, Alkylreste, substituiert durch Hydroxy, Amino oder Alkylamino, bedeuten, und daß man dann gegebenenfalls das erhaltene Produkt in seine Isomeren auftrennt.

**Anspruch** (für den Vertragsstaat/AT)

Verfahren zur Herstellung eines 3-Vinyl-cephalosporin-Derivats der allgemeinen Formel :

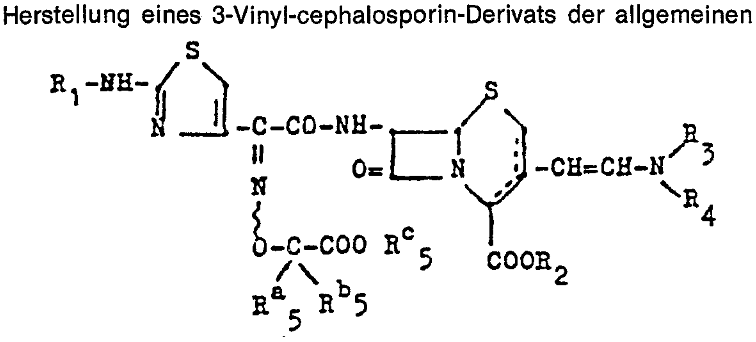

welches in Bicycloocten-2 oder -3-Form vorliegt und worin
— der Substituent in 3-Stellung des Bicyclooctens die E- oder Z-Stereoisomerie aufweist ;
— die Iminogruppe des Substituenten in 7-Stellung in syn- oder anti-Form vorliegt ;
— die Reste $R^a_5$ und $R^b_5$, welche identisch oder verschieden sind, Wasserstoffatome oder Alkylreste darstellen oder zusammen einen Alkylenrest mit 2 oder 3 Kohlenstoffatomen bilden ;
— das Symbol $R^c_5$ einen Säureschutzrest bedeutet ;
— das Symbol $R_1$ einem Aminschutzrest bedeutet ;
— das Symbol $R_2$ einen auf enzymatischem Wege leicht abspaltbaren Rest der allgemeinen Formel :

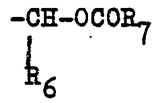

⟦worin $R_6$ ein Wasserstoffatom oder einen Alkylrest und $R_7$ einen Alkylrest oder den Cyclohexylrest bedeutet⟧ oder einen Methoxymethyl-, tert.-Butyl-, Benzhydryl-, p-Nitrobenzyl- oder p-Methoxybenzylrest bedeutet ; und
— die Symbole $R_3$ und $R_4$, welche identisch oder verschieden sind, Alkylreste (gegebenenfalls substituiert durch einen Hydroxy-, Alkyloxy-, Amino-, Alkylamino- oder Dialkylaminorest) oder Phenyl bedeuten oder zusammen mit dem Stickstoffatom, an das sie gebunden sind, einen gesättigten Heterocyclus mit 5 oder 6 Kettengliedern, enthaltend gegebenenfalls ein weiteres Heteroatom, ausgewählt unter Stickstoff, Sauerstoff oder Schwefel, und gegebenenfalls substituiert durch einen Alkylrest, bilden, woben die oben erwähnten Alkylteile oder -reste (wenn nichts anderes angegeben ist) geradkettig oder verzweigt sind und 1 bis 4 Kohlenstoffatome enthalten, sowie von den Gemischen ihrer Isomeren, dadurch gekennzeichnet, daß man zur Herstellung eines Produktes, in dessen Formel $R_3$ und $R_4$ wie oben definiert sind, mit Ausnahme der Bedeutung Alkyl, substituiert durch Hydroxy, Amino oder Alkylamino, ein Produkt der allgemeinen Formel :

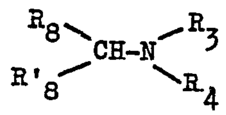

[worin $R_3$ und $R_4$ wie oben definiert sind und $R_8$ und $R'_8$, welche identisch oder verschieden sind, entweder Gruppen der allgemeinen Formel :

$$—X_2R_9$$

bedeuten, worin $X_2$ Sauerstoff ist und $R_9$ ein Alkylrest mit 1 bis 5 Kohlenstoffatomen oder Phenyl ist, oder entweder jeweils einen Aminorest der allgemeinen Formel :

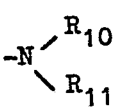

bedeuten, worin $R_{10}$ und $R_{11}$ wie $R_3$ und $R_4$ definiert sind, oder auch einer einen Rest $—X_2R_9$, worin $X_2$ Sauerstoff oder Schwefel ist, und der andere einen Rest $—NR_{10}R_{11}$ bedeutet] mit einem Derivat des Cephalosporins der allgemeinen Formel :

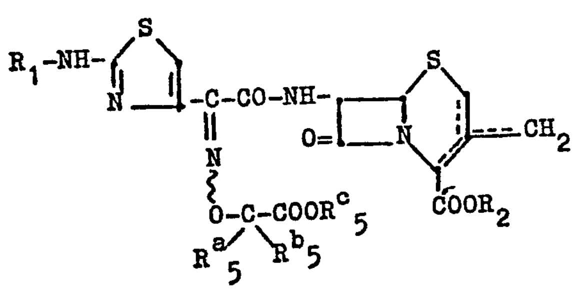

umsetzt, welche in Form Bicycloocten-2 oder -3 oder 3-Methylen-bicyclooctan vorliegt und worin die verschieden Symbole wie oben definiert sind, und daß man dann gegebenenfalls das erhaltene Produkt in seine Isomeren auftrennt ; oder daß man zur Herstellung eines Produktes, in dessen Formel $R_3$ und $R_4$,

welche identisch oder verschieden sind, Alkylreste, substituiert durch Hydroxy, Amino oder Alkylamino. bedeuten, die Trans-Enaminierung eines vorstehend erhaltenen Produktes [wobei $R_3$ und $R_4$ Alkylreste bedeuten und die anderen Symbole wie oben definiert sind] durch Einwirkung eines Amins der allgemeinen Formel :

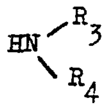

bewirkt, worin $R_3$ und $R_4$, welche identisch oder verschieden sind, Alkylreste, substituiert durch Hydroxy, Amino oder Alkylamino, bedeuten, und daß man dann gegebenenfalls das erhaltene Produkt in seine Isomeren auftrennt.